# EUROPEAN PATENT APPLICATION

(11) **EP 1 777 519 A1**
(43) Date of publication of application: **25.04.2007**
(21) Application number: 06118663.1
(22) Date of filing: 31.10.2002
(51) Int. Cl.: G01N 33/50, A61K 38/18, A61K 45/06, C12Q 1/68, C07K 14/51

(54) **Bone morphogenic proteins (BMP), BMP receptors and BMP binding proteins and their use in the diagnosis and treatment of glaucoma**

(30) Priority: 31.10.2001 US 334852 P
(62) Divisional of application: 02802562.5
(71) Applicant: Alcon, Inc., 6221 Hünenberg (CH); UNIVERSITY OF NORTH TEXAS HEALTH SCIENCE CENTER, Office of Research, Fort Worth, TX 76107 (US)
(72) Inventor: Clark, Abbot F., Arlington, TX 76017 (US); Wordinger, Robert J., Euless, TX 76017 (US)
(74) Representative: Moore, Barry

(57) **Abstract**

The present invention provides methods for identifying an agent useful for the treatment of glaucoma.

## Description

### 1. Field of the Invention

The present invention discloses methods and reagents for diagnosing and treating glaucoma and related disorders.

### 2. Description of the Related Art

"Glaucomas" are a group of debilitating eye diseases that are the leading cause of irreversible blindness in the United States and other developed nations. Primary Open Angle Glaucoma ("POAG"), the most common form of glaucoma, is characterized by the degeneration of the trabecular meshwork, leading to obstruction of the normal ability of aqueous humor to leave the eye without closure of the space (e.g., the "angle") between the iris and cornea (Vaughan, D. *et al.,* (1992)). A characteristic of such obstruction in this disease is an increased intraocular pressure ("IOP"), resulting in progressive visual loss and blindness if not treated appropriately and in a timely fashion. The disease is estimated to affect between 0.4% and 3.3% of all adults over 40 years old (Leske, M. C. *et al.* (1986); Bengtsson, B. (1989); Strong, N. P. (1992)). Moreover, the prevalence of the disease rises with age to over 6% of those 75 years or older (Strong, N. P., (1992)).

Because increased IOP is a readily measurable characteristic of glaucoma, the diagnosis of the disease is largely screened for by measuring intraocular pressure (tonometry) (Strong, N. P. (1992); Greve, M. *et al.* (1993)). Unfortunately, because glaucomatous and normal pressure ranges overlap, such methods are of limited value unless multiple readings are obtained (Hitchings, R. A., (1993); Tuck, M. W. *et al.* (1993); Vaughan, D. *et al.,* (1992); Vemon, S. A., (1993)). For this reason, additional methods, such as direct examination of the optic disk and determination of the extent of a patient's visual field loss are often conducted to improve the accuracy of diagnosis (Greve, M. *et al.,* (1993)).

Glaucoma affects three separate tissues in the eye. The elevated IOP associated with POAG is due to morphological and biochemical changes in the trabecular meshwork (TM), a tissue located at the angle between the cornea and iris. Most of the nutritive aqueous humor exits the anterior segment of the eye through the TM. The progressive loss of TM cells and the build-up of extracellular debris in the TM of glaucomatous eyes leads to increased resistance to aqueous outflow (Lutjen-Drecoll and Rohen 1996; Rohen 1983; Rohen *et al.* 1993; Grierson and Calthorpe 1988), thereby raising IOP. Elevated IOP, as well as other factors such as ischemia, cause degenerative changes in the optic nerve head (ONH) leading to progressive "cupping" of the ONH (Varma and Minckler 1996; Hernandez and Gong 1996; Hernandez *et al.* 1990; Hernandez and Pena 1997; Morrison *et al.* 1990) and loss of retinal ganglion cells (Quigley *et al.* 2000; Quigley 1999; Quigley *et al.* 1995; Kerrigan *et al.* 1997) and axons. The detailed molecular mechanisms responsible for glaucomatous damage to the TM, ONH, and the retinal ganglion cells are unknown.

Current glaucoma therapy is directed to lowering IOP, a major risk factor for the development and progression of glaucoma. These therapies lower IOP, but they do not directly address the pathogenic mechanisms, and the disease continues to progress. At least half of patients with glaucoma are undiagnosed, and by the time patients are diagnosed with glaucoma, they have already lost approximately 40% of their retinal ganglion cells. Therefore, methods for earlier detection and diagnosis of glaucoma are needed.

In view of the importance of glaucoma, and the at least partial inadequacies of prior methods of diagnosis, it would be desirable to have an improved, more accurate method for diagnosing glaucoma in its early stages. In addition, it would be desirable to have new therapeutic agents that address glaucomatous pathogenic mechanisms.

### SUMMARY OF THE INVENTION

The present invention overcomes these and other drawbacks of the prior art by providing methods and kits for the early diagnosis of glaucoma, for treating glaucoma, and for the identification of compounds useful in the treatment of glaucoma.

In certain specific embodiments, the invention provides a method for diagnosing glaucoma in a sample obtained from a cell or bodily fluid by detecting altered expression of a bone morphogenic protein family member gene. The method generally includes the steps of:
a) obtaining a tissue or fluid sample from a patient suspected of having glaucoma;
b) extracting DNA from said sample;
c) obtaining a plurality of PCR primers, wherein said primers each comprise a sequence consisting of from 18 to 1547 contiguous nucleotides from SEQ ID NO:1, SEQ ID NO:3, SEQ ID NO:5, SEQ ID NO:7, SEQ ID NO: 37, SEQ ID NO:39, SEQ ID NO:41, SEQ ID NO:43, SEQ ID NO:45, SEQ ID NO:47, or SEQ ID NO:53;
d) amplifying regions of the extracted DNA using said primers to obtain a PCR product;
e) resolving the PCR product; and
f) identifying differences between the sequence of the PCR product and the normal gene sequence;
where a difference between the amplified sequence and the normal gene sequence is diagnostic of glaucoma.

In general, the methods of the invention may include obtaining a sample from an individual and extracting DNA from said sample. Select PCR primers for specific members of the BMP gene family are then used to amplify relevant regions of the extracted gene to obtain a PCR product. The PCR product is resolved by a technique that effectively identifies DNA sequence differences between the normal and mutated form of the specific BMP family gene being evaluated (the extracted DNA). Identified differences between the sequences are indicative of glaucoma.

The tissue or fluid sample for use in the methods of the invention may be blood or buccal cells.

Typically, the primer sequences will have a length of between about 10, 15 or 18 nucleotides to about 20, or to about 30 nucleotides. Longer sequences, e.g., 40, 50, 80, 90, 95, 100, even up to full length, are even more preferred for certain embodiments. Lengths of oligonucleotides of at least about 18 to 20 nucleotides are well accepted by those of skill in the art as sufficient to allow sufficiently specific hybridization so as to be useful as a molecular probe, as described by Lathe (1985), which reference is specifically incorporated herein by reference for this purpose. Preferably, the nucleotide sequence will consist of from 20 to 100 contiguous nucleotides of SEQ ID NO:1, SEQ ID NO:3, SEQ ID NO:5, SEQ ID NO:7, SEQ ID NO:37, SEQ ID NO:39, SEQ ID NO:41, SEQ ID NO:43, SEQ ID NO:45, SEQ ID NO:47, or SEQ ID NO:53. It is also contemplated that the primer sequences may consist of sequences of at least 10, 15 or 18 contiguous nucleotides from the sequences of BMP receptor genes and from BMP-associated proteins, the sequences of which are known.

Nucleic acid molecules having stretches of 10, 18, 20, 30, 50, 60, 65 or even up to and including 100 nucleotides or so, complementary to any one of SEQ ID NO: 1, SEQ ID NO:3, SEQ ID NO:5, SEQ ID NO:7, SEQ ID NO:37, SEQ ID NO:39, SEQ ID NO:41, SEQ ID NO:43, SEQ ID NO:45, SEQ ID NO:47, or SEQ ID NO:53, have utility as hybridization probes. Primers or probes having a nucleotide length of about 18 nucleotides are recognized by those of skill in the art to provide highly specific hybridization to a target sequence. The total size of the fragment, as well as the size of the complementary stretches, will ultimately depend on the intended use of application of the particular nucleic acid segment. Smaller fragments will generally find use in hybridization embodiments, wherein the length of the complementary region may be varied, such as between about 10, 18, 20 or 30 and about 50, 60, 70, 80, 80 or 100 nucleotides, or even full length according to the complementary sequences one wishes to detect.

In specifically preferred embodiments, the primers will consist of contiguous sequences from SEQ ID NO: 1, SEQ ID NO:3, SEQ ID NO:5, SEQ ID NO:7, SEQ ID NO:37, SEQ ID NO:39, SEQ ID NO:41, SEQ ID NO:43, SEQ ID NO:45, SEQ ID NO:47, or SEQ ID NO:53. In other preferred embodiments, the primers will consist of contiguous sequences from BMP receptor genes (disclosed in ten Dijke *et al.* 1993; Astrom *et al.* 1999; Nohno *et al.* 1995, all incorporated herein by reference) or from BMP-associated genes, such as chordin (NCBI NM_029130), gremlin (Murphy *et al.* 1999; McMahon *et al.* 2000), follistatin (NCBI NM_003892) or bambi (NCBI NM_005791). Most preferably, the primers will consist of contiguous sequence from SEQ ID NO:3. In certain aspects, at least some of the primers may further include a detectable label.

In other embodiments, the invention provides a method for treating glaucoma by administering to a patient in need thereof a composition comprising a sequence consisting of at least one compound selected from the group consisting of a BMP2 agonist, a BMP4 agonist, a BMP5 agonist, a BMP7 agonist, a Smad 1/5 agonist, a chordin antagonist, a gremlin antagonist and a follistatin antagonist.

In additional aspects, the present invention provides a method for identifying a therapeutic agent for the treatment of glaucoma. Therapeutic agents may be identified, for example, by:
a) obtaining a first composition comprising a population of recombinant cells expressing BMP-2A, BMP4, BMP-5, or BMP7;
b) obtaining a candidate substance;
c) incubating said composition and said candidate substance;
testing said composition for its ability to turn on BMP-induced Smad signaling pathways and/or BMP-regulated gene expression; and identifying a candidate substance that inhibits, or stimulates, these downstream effects of BMP.

Another aspect of the invention are diagnostic kits containing sequences of the present invention and suitable reagents such as a detectable label linked to a protein, peptide or the antibody itself Alternatively, the detectable label may be linked to a second sequence which selectively hybridizes to a sequence of the invention.

Related embodiments include therapeutic kits which include pharmaceutically-acceptable formulations of either the nucleic acid sequences or peptide or protein sequences disclosed herein. Such kits are useful in the detection of altered expression of the BMP genes and proteins in clinical samples for the diagnosis of glaucoma.

### BRIEF DESCRIPTION OF THE DRAWINGS

The drawings form part of the present specification and are included to further demonstrate certain aspects of the present invention. The invention may be better understood by reference to one or more of these drawings in combination with the detailed description of specific embodiments presented herein.

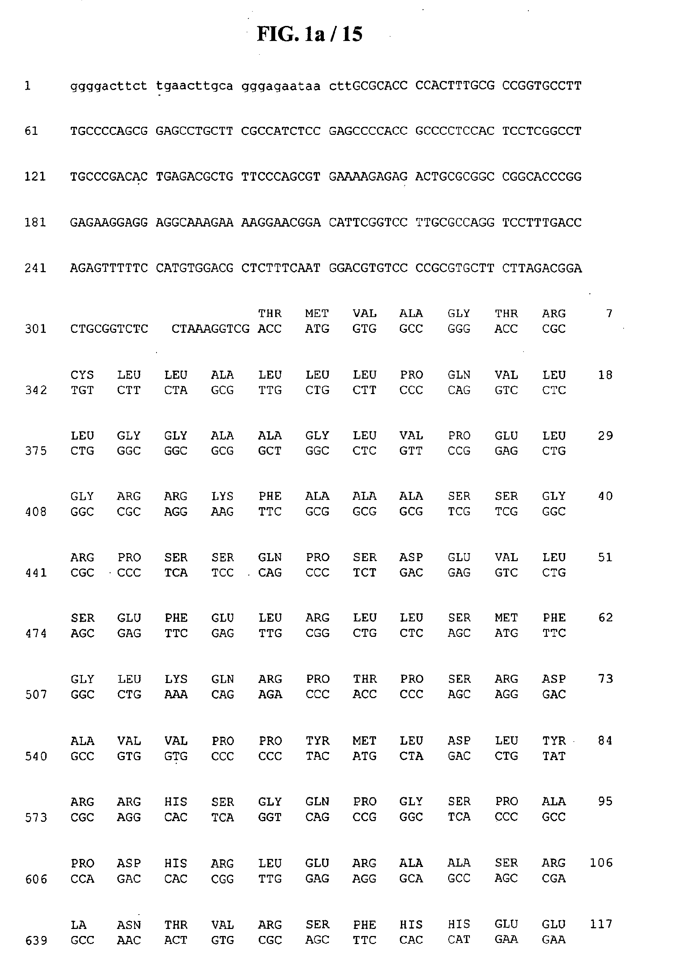

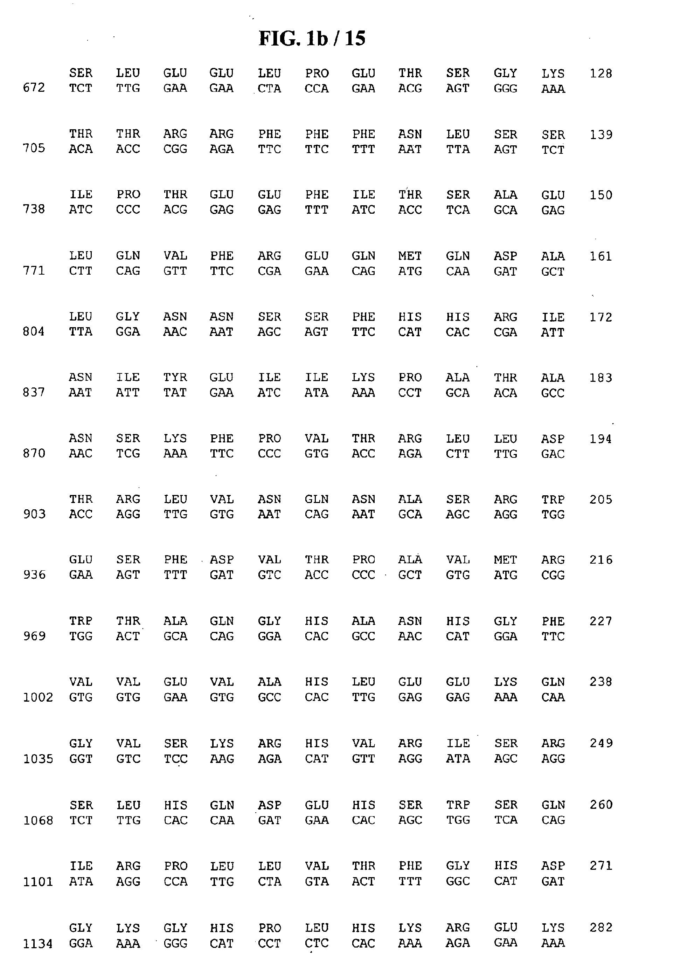

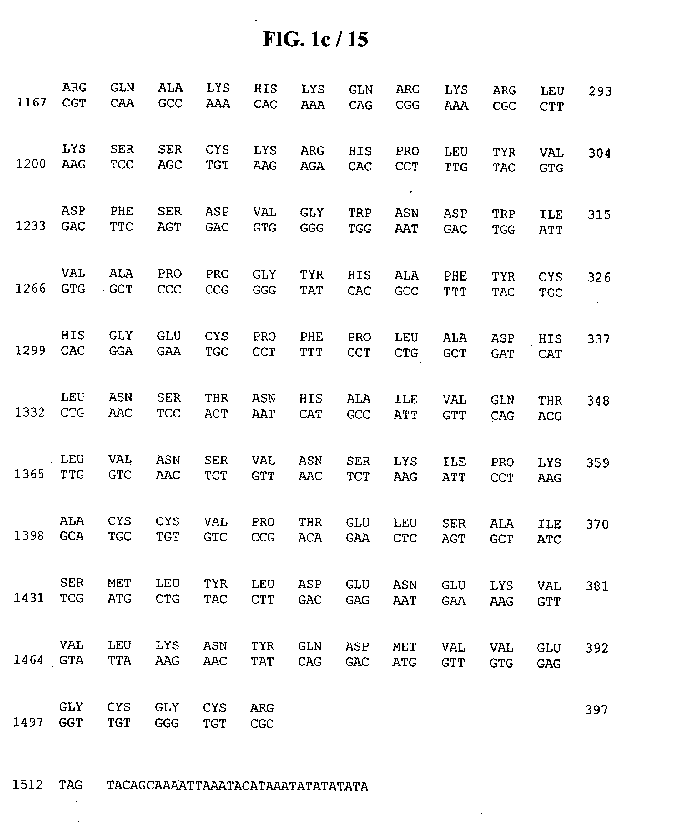

FIG. 1. Nucleotide and amino acid sequence of BMP2A.

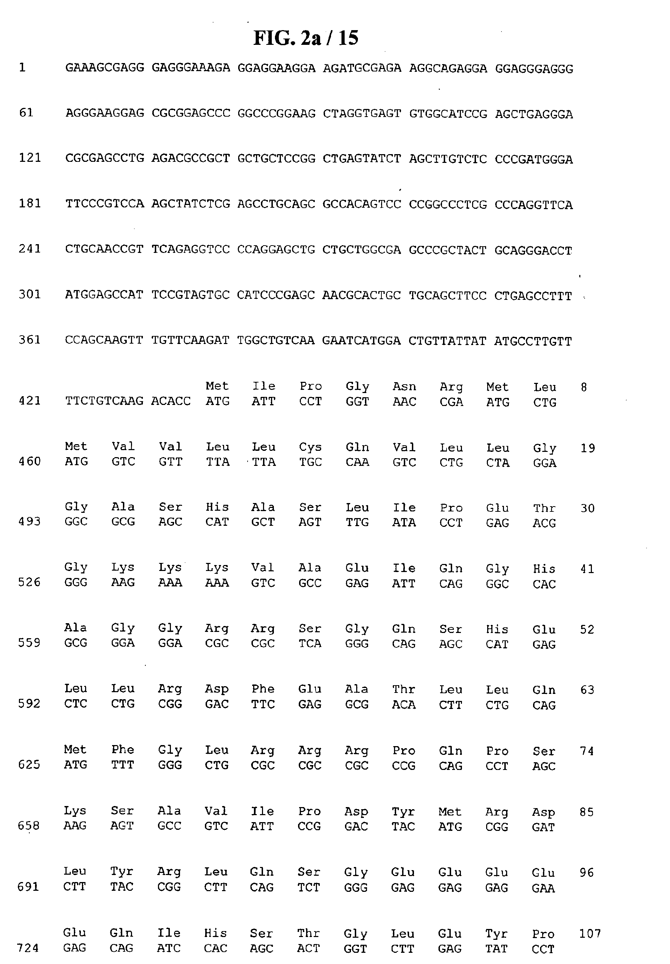

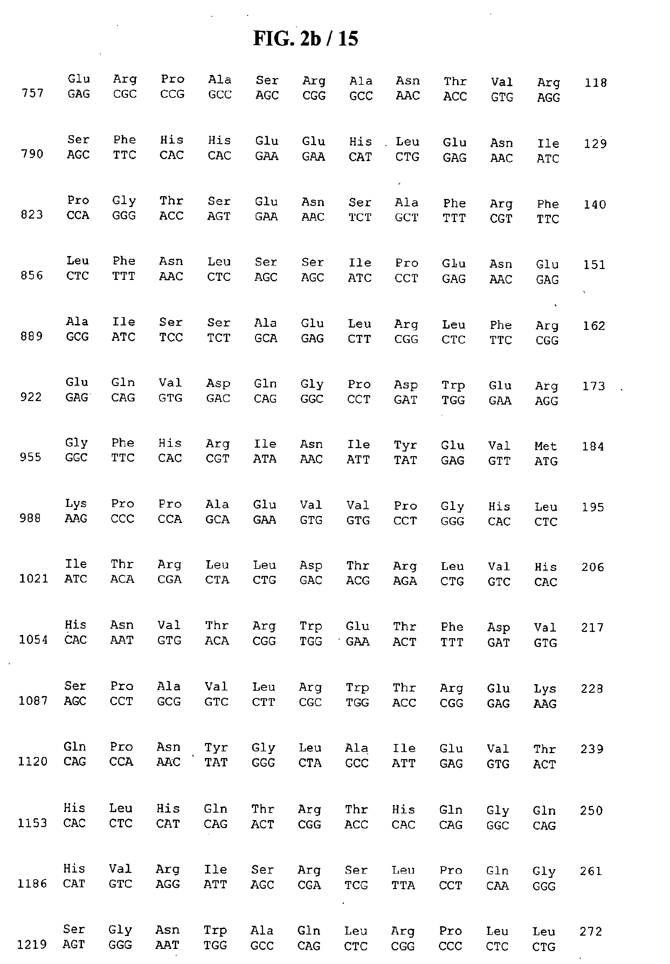

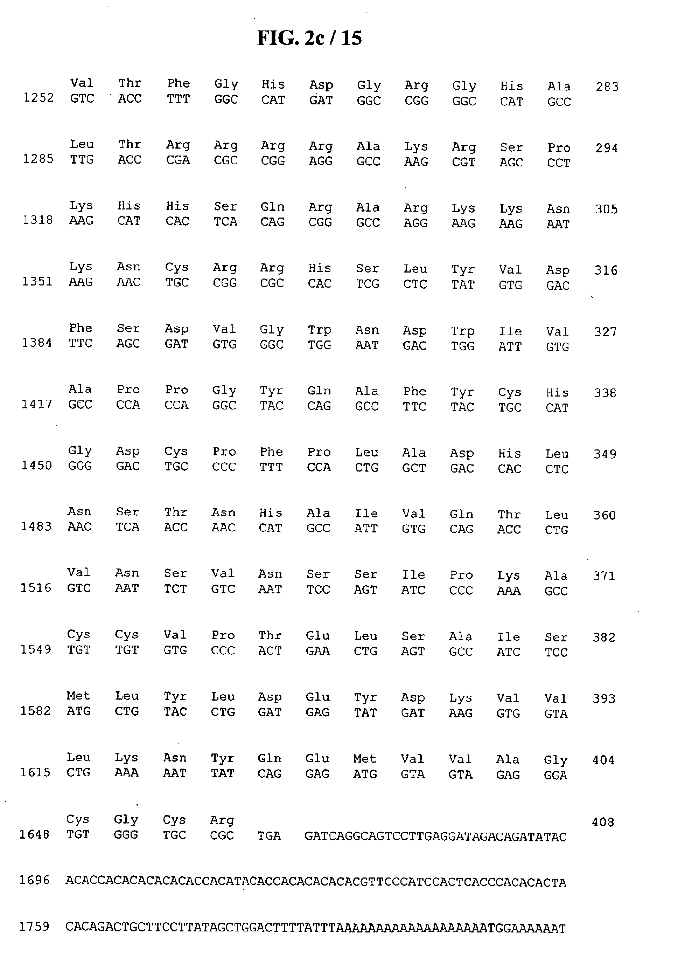

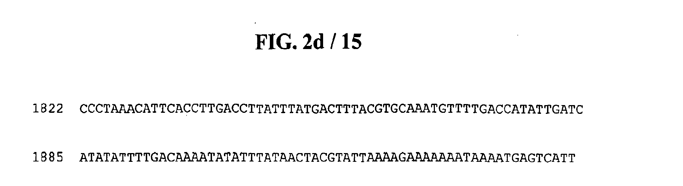

FIG.2. Nucleotide and amino acid sequence of BMP4.

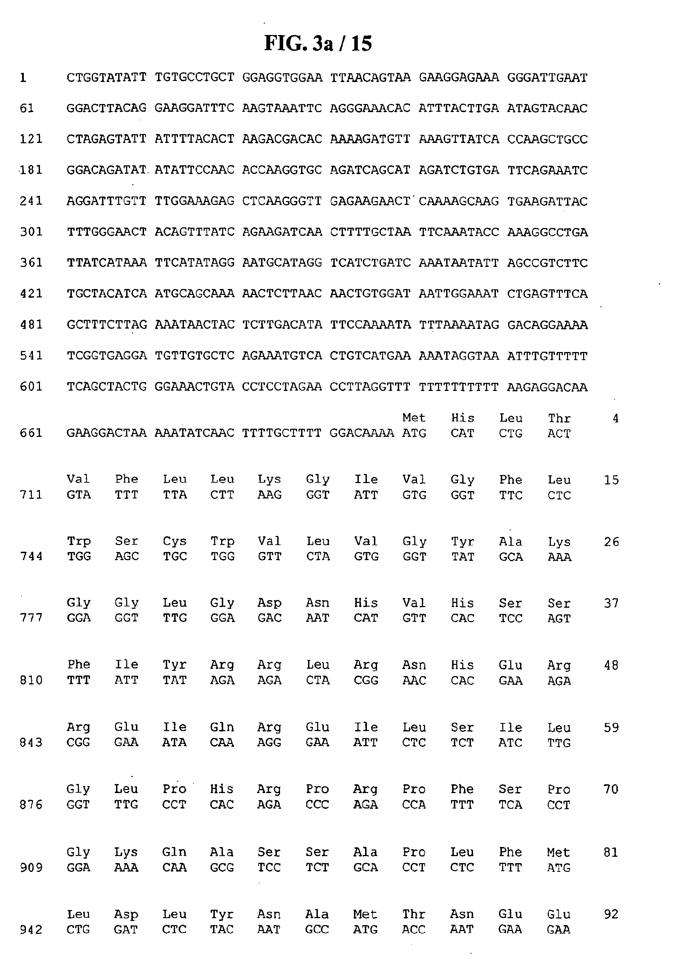

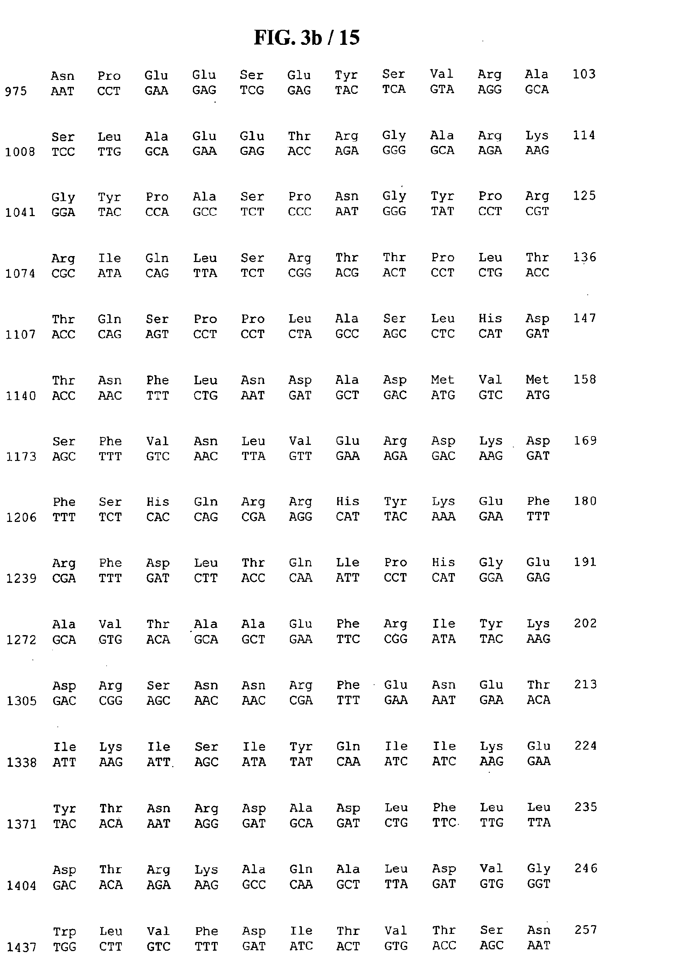

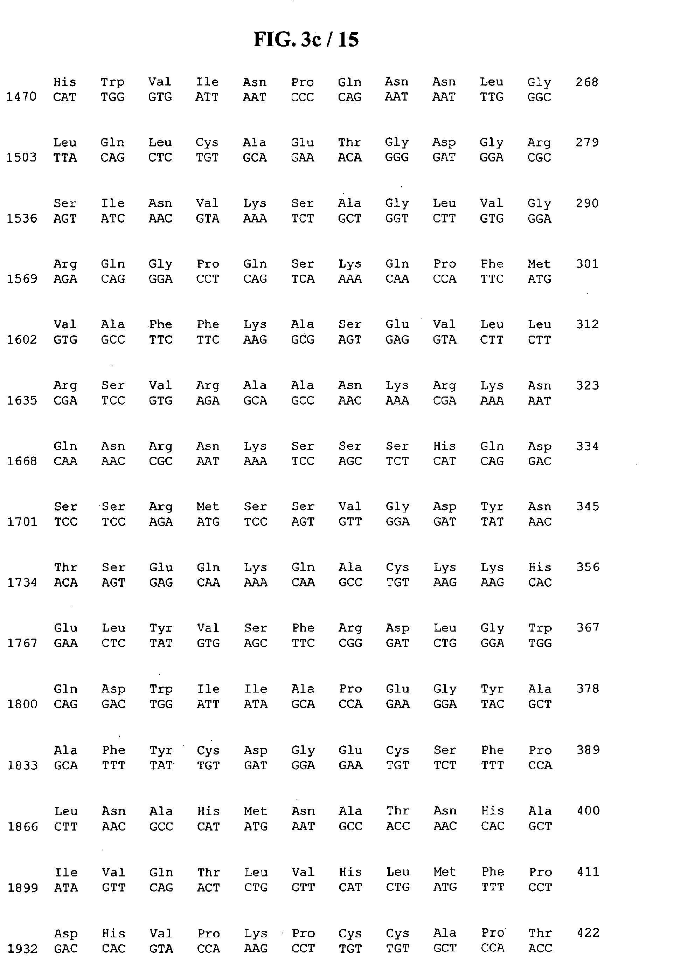

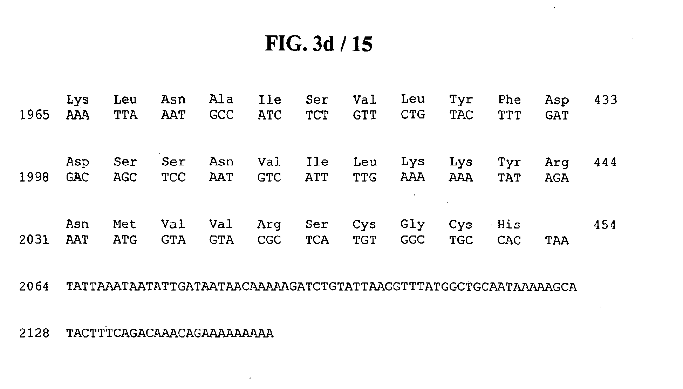

FIG. 3. Nucleotide and amino acid sequence of BMP5.

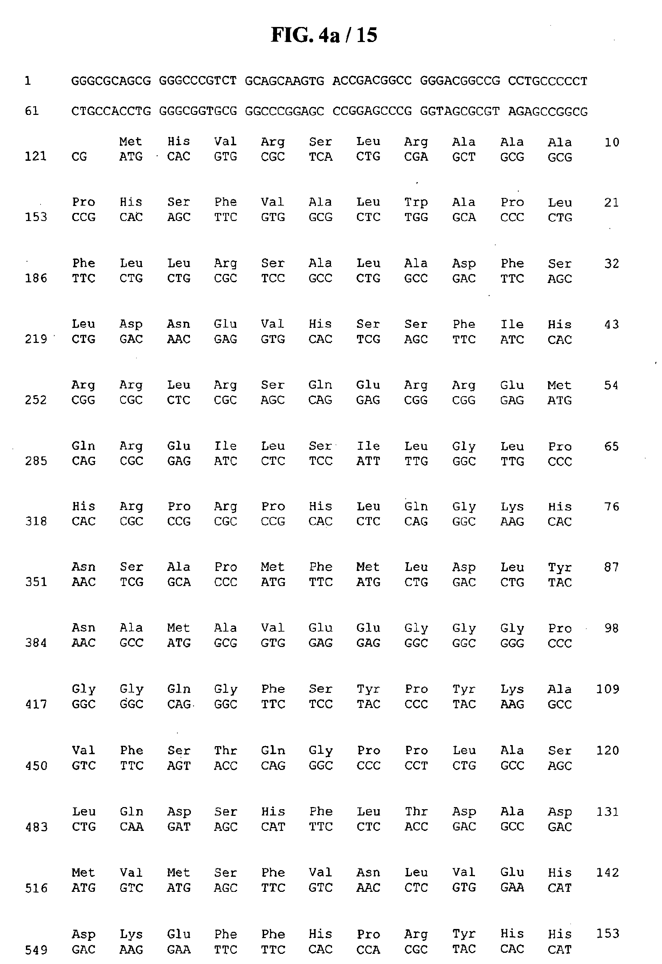

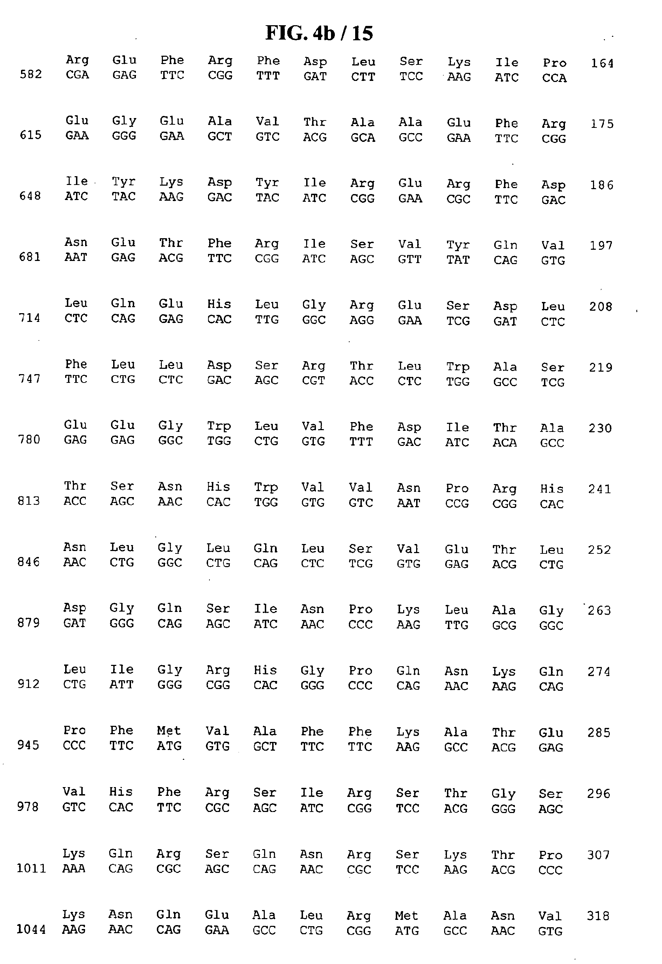

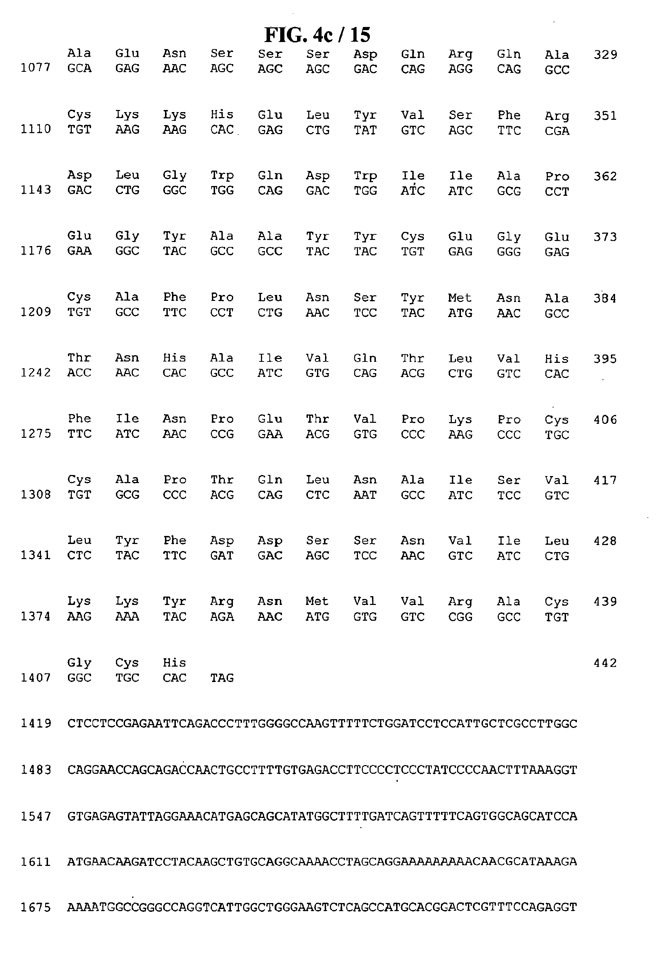

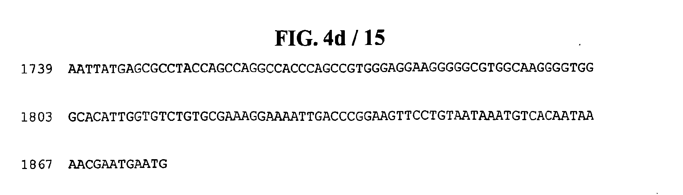

FIG. 4. Nucleotide and amino acid sequence of BMP7.

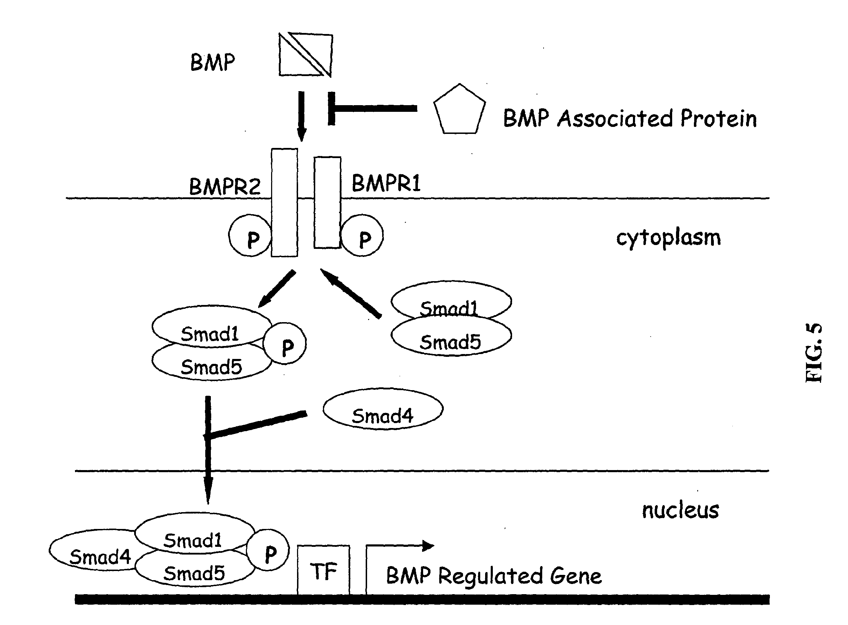

FIG. 5. Bone morphogenic protein signaling pathway. Bone Morphogenic Protein (BMP) dimers bind to a membrane complex composed of BMP receptors 1 and 2, which are serin/threonine kinases. The regulatory Smads (Smad1/Smad5) become phosphorylated and associate with a co-Smad (Smad 4). This resulting Smad complex enters the nucleus where it associates with transcription factors (TF) and regulates gene expression. BMP associated proteins act as BMP antagonists by binding BMPs and preventing BMP interaction with BMP receptors.

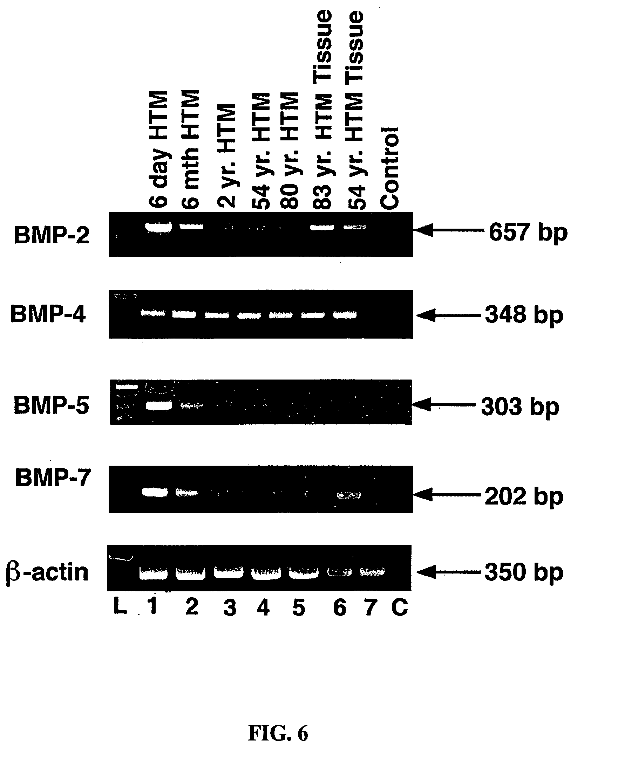

FIG. 6. BMP expression in human TM cells and tissues. Ethidium bromide-stained agarose gel of BMP PCR products from cDNA samples generated from RT-PCR analysis of BMP expression in human TM cells (lanes 1-5) and tissues (lanes 6-7). L = base pair markers. C = PCR negative control lane. β-actin was used as a positive RT-PCR internal control.

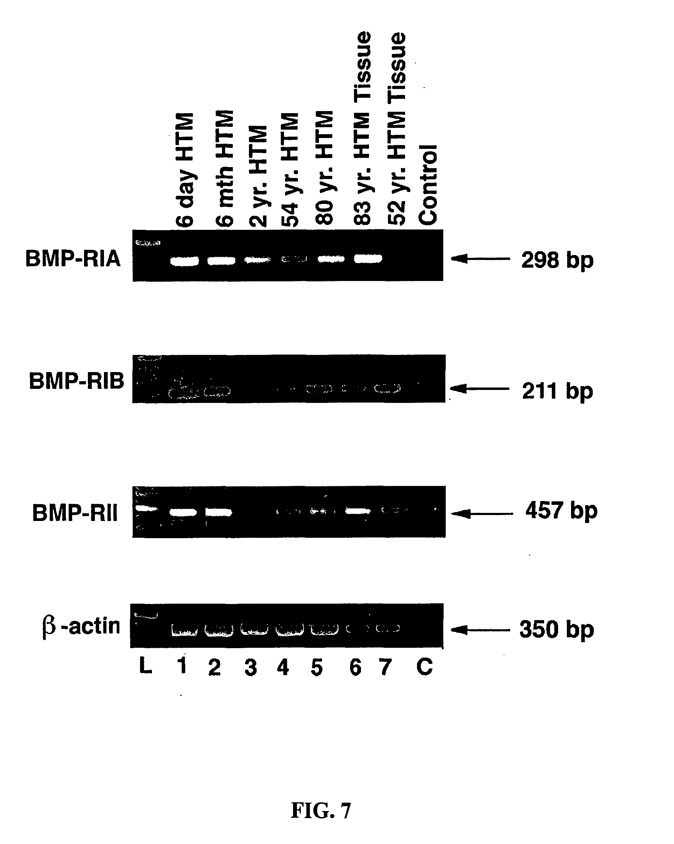

FIG. 7. BMP receptor expression in human TM cells and tissues. Ethidium bromide-stained agarose gel of PCR products from cDNA samples generated from RT-PCR analysis of BMP receptor expression in human TM cells (lanes 1-5) and tissues (lanes 6-7). L = base pair markers. C = PCR negative control lane. β-actin was used as a positive RT-PCR internal control.

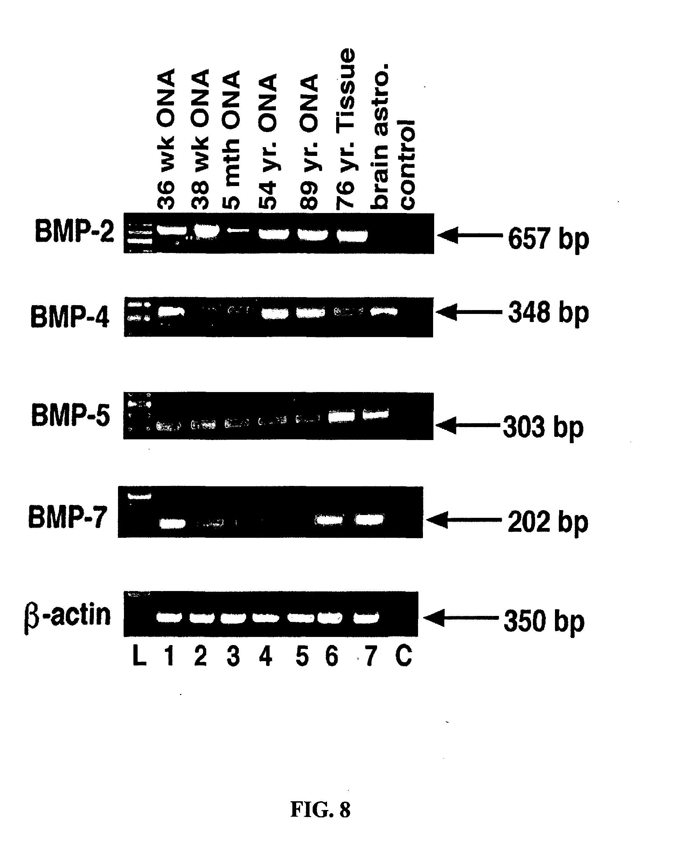

FIG. 8. BMP expression in human ONH astrocytes, ONH tissues, and human brain astrocytes. Ethidium bromide-stained agarose gel of PCR products from cDNA samples generated from RT-PCR analysis of BMP expression in human ONH astrocyte (lanes 1-5), ONH tissue (lane 6), and human brain astrocytes (lane 7). L = base pair markers. C = PCR negative control lane. β-actin was used as a positive RT-PCR internal control.

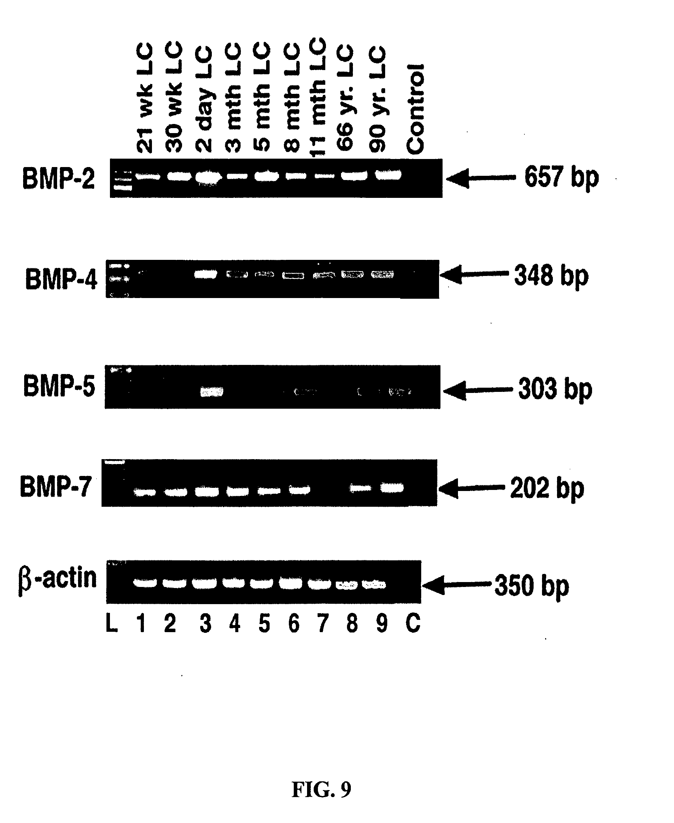

FIG. 9. BMP expression in human lamina cribrosa cell lines. Ethidium bromide-stained agarose gel of PCR products from cDNA samples generated from RT-PCR analysis of human lamina cribrosa cells (lanes 1-9). L = base pair markers. C = PCR negative control lane. β-actin was used as a positive RT-PCR internal control.

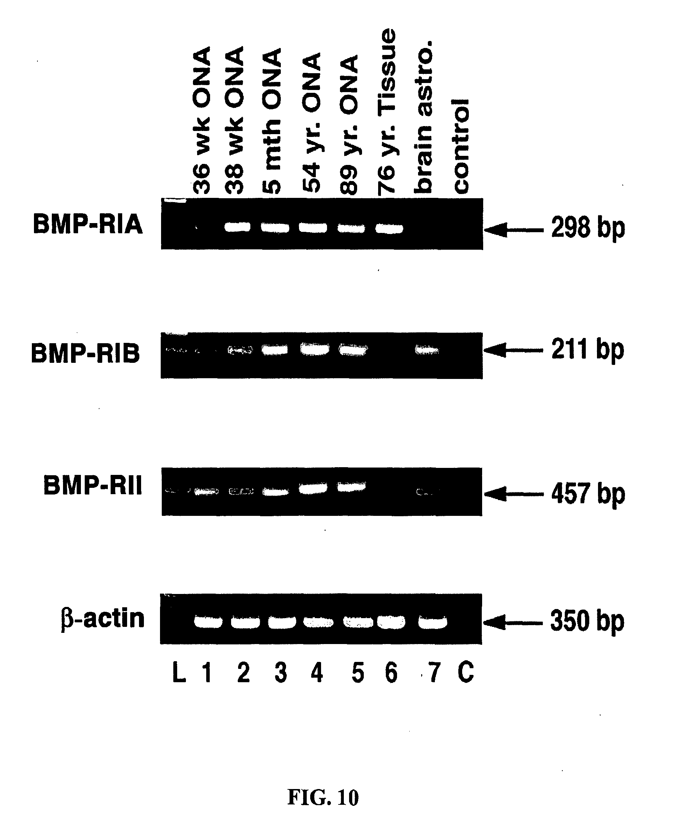

FIG. 10. BMP receptor expression in human ONH astrocytes, ONH tissues, and human brain astrocytes. Ethidium bromide-stained agarose gel of PCR products from cDNA samples generated from RT-PCR analysis of BMP receptor expression in human optic nerve head astrocytes (ONA) (lanes 1-5), ONH tissue (lane 6), and human brain astrocytes (lane 7). L = base pair markers. C = PCR negative control lane. β-actin was used as a positive RT-PCR control.

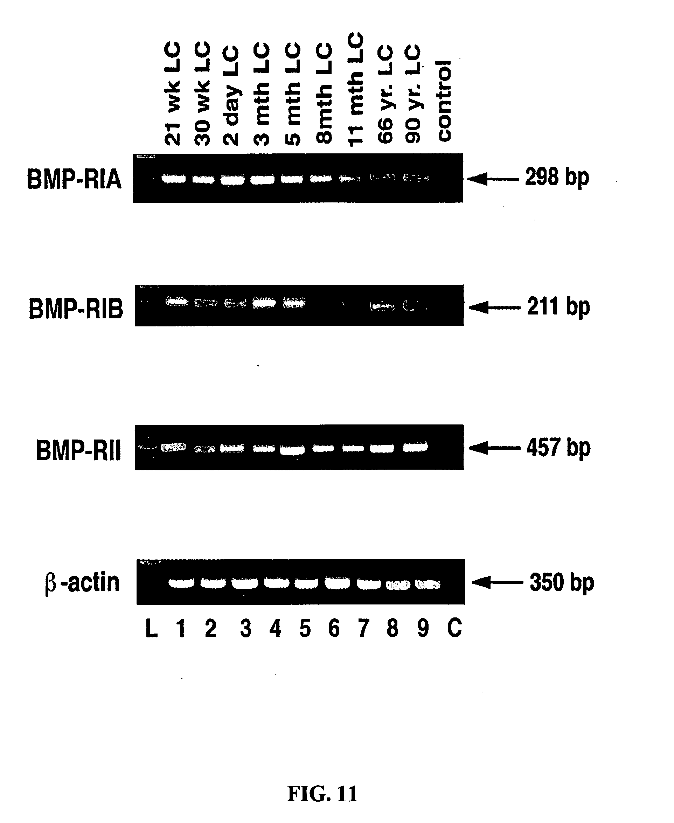

FIG. 11. BMP receptor expression in human lamina cribrosa cell lines. Ethidium bromide-stained agarose gel of PCR products from cDNA samples generated from RT-PCR analysis of human lamina cribrosa cells (lanes 1-9). L = base pair markers. C = PCR negative control lane. β-actin was used as a positive RT-PCR control.

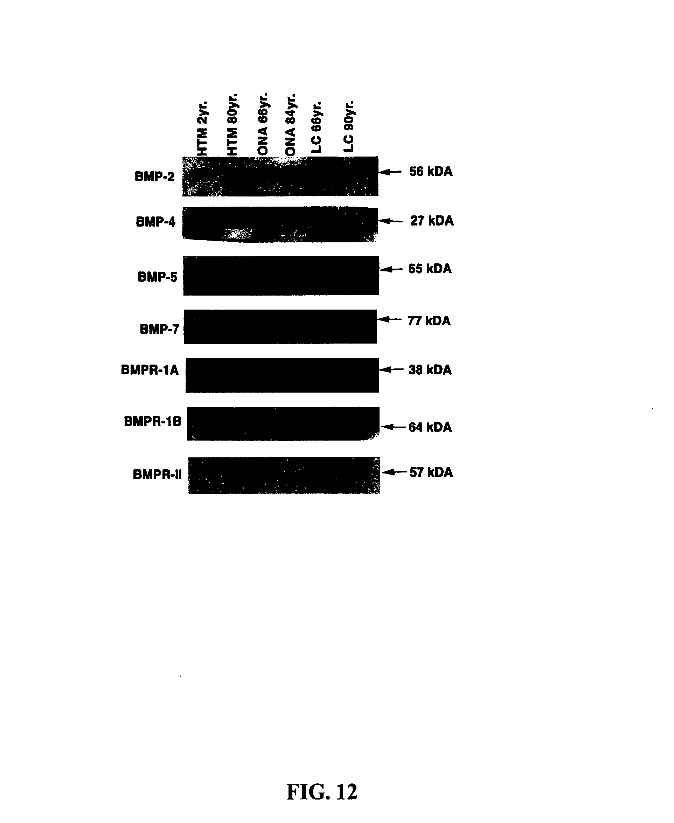

FIG. 12. Western immunoblot of BMP and BMP receptor expression in cultured human TM cells, optic nerve head astrocytes (ONA), and lamina cribrosa cells. Chemiluminescent detection of BMP proteins and BMP receptors in human trabecular meshwork cells (lanes 1-2), ONH astrocytes (lanes 3-4), and lamina cribrosa cells (lanes 5-6). Protein size indicated in kDa.

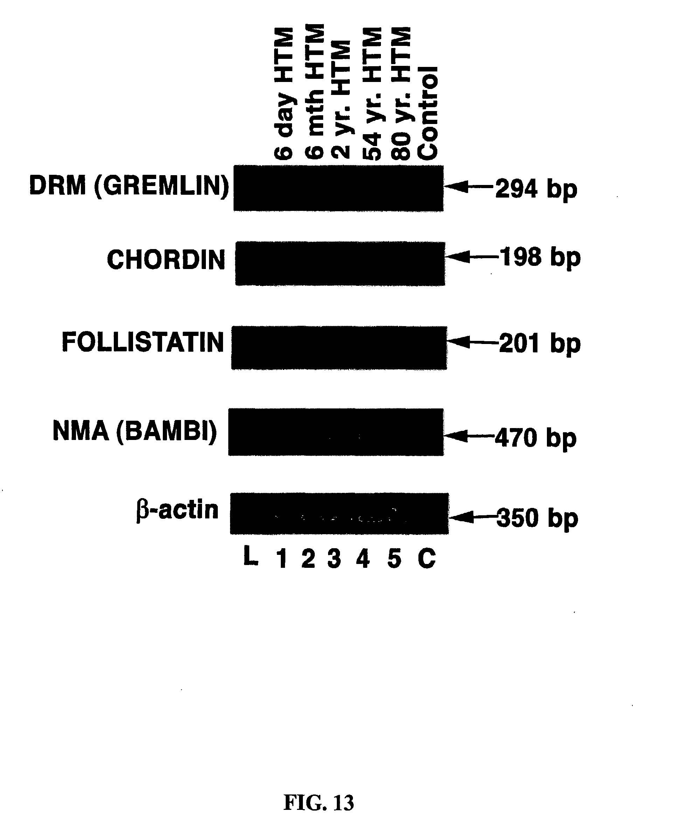

FIG. 13. BMP associated protein mRNA expression in human TM cells. Ethidium bromide-stained agarose gel of PCR products from cDNA samples generated from RT-PCR analysis of human TM cells (lanes 1-5). L = base pair markers. C = PCR negative control lane. β-actin was used as a positive RT-PCR internal control.

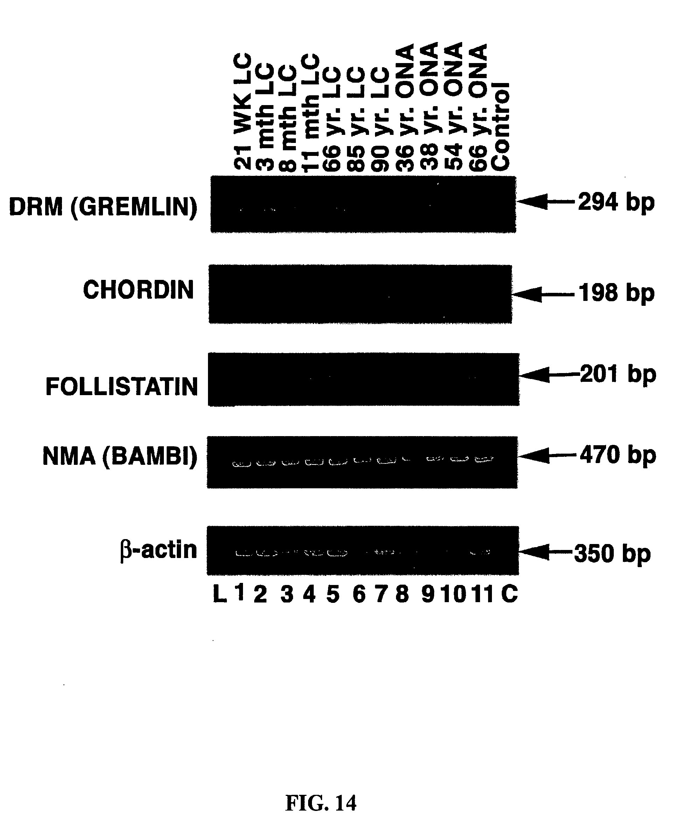

FIG. 14. BMP associated protein mRNA expression in human lamina cribrosa cells and ONH astrocytes. Ethidium bromide-stained agarose gel of PCR products from cDNA samples generated from RT-PCR analysis of lamina cribrosa (LC) cells (lanes 1-7) and ONH astrocytes (ONA) (lanes 8-11). L = base pair markers. C = PCR negative control lane. β-actin was used as a positive RT-PCR internal control.

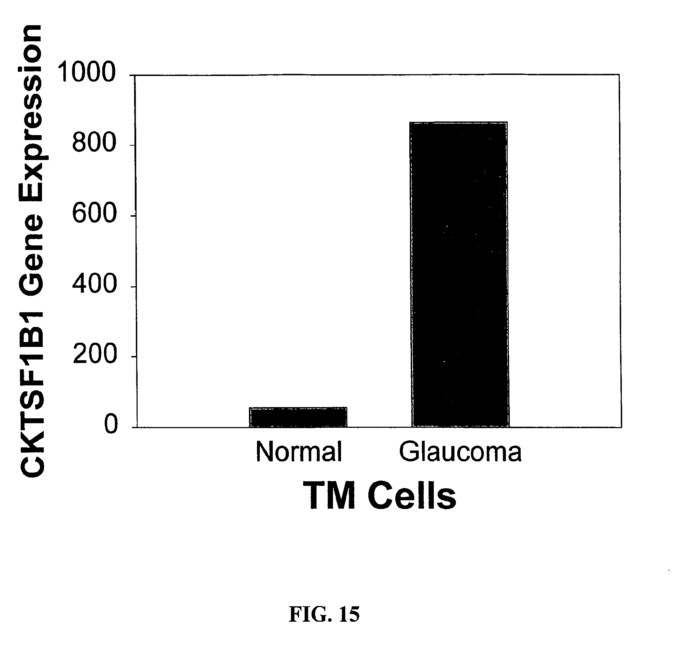

FIG. 15. Illustrates increased expression of the BMP antagonist gremlin (CKTSF1B1) in glaucomatous TM cells. Gene expression was assessed using Affymetrix gene arrays (Affymetrix gene chip U133A).

### DETAILED DESCRIPTION PREFERRED EMBODIMENTS

The trabecular meshwork has been proposed to play an important role in the normal flow of the aqueous humor, and has been presumed to be the major site of outflow resistance in glaucomatous eyes. Human trabecular meshwork (HTM) cells are specialized cells which line the outflow channels by which aqueous humor exits the eye. Altered synthetic function of the cells may be involved in the pathogenesis of POAG, steroid glaucoma, and other types of glaucoma.

Despite years of intensive research, the precise molecular mechanisms responsible for glaucomatous damage to the eye are not known. Recent research has suggested that growth factors may be important in maintaining normal homeostasis in the ocular tissues associated with glaucoma, and alterations in growth factor/growth factor receptors may play a role in glaucoma pathogenesis. Growth factors area very large family of polypeptides that control cell growth and differentiation. These molecules have a variety of cell-specific effects on gene expression, extracellular matrix composition and deposition, cytoskeletal organization, and regulation of cellular functions. The TM expresses a wide variety of growth factors, growth factor receptors (Tripathi *et al.* 1993a; Tripathi *et al.* 1993b; Tripathi *et al..* 1994a; Tripathi *et al.* 1994b; Wordinger *et al.* 1998; Wordinger *et al.* 1999) as well as neurotrophin/neurotrophic factors and their receptors (Liu *et al.* 2001; Wordinger *et al.* 2000). ONH astrocytes and lamina cribrosa cells, two cell types of the optic nerve head, express growth factors, neurotrophins and their receptors (Lambert *et al.* 2001; Pena *et al.* 1999). The aqueous humor also contains a variety of growth factors including FGF2, EGF, TGFβ, HGF (Tripathi *et al.* 1996; Tripathi *et al.* 1991; Tripathi *et al.* 1992; Hu and Ritch 2001) as well as neurotrophins (Chundru *et al.* 2000). Elevated levels of aqueous humor TGFβ-2 and HGF have been reported in POAG patients (Tripathi *et al.* 1994c; Inatani *et al.* 2001; Picht *et al.* 2001). Growth factors may be involved in glaucoma by altering the normal development and/or function of the TM and ONH.

The present invention stems in part from the recognition that bone morphogenic proteins (BMPs) not only induce bone and cartilage formation but are multifunctional cytokines having a wide range of effects on numerous cell types (Hogan 1996; Reddi 1997) and are expressed by both human trabecular meshwork (HTM) and optic nerve head (ONH) cells (Wordinger *et al.* 2002). BMPs are members of the TGFβ superfamily, and there are approximately 15-20 BMPs genes in man, 3 BMP receptors, and a number of BMP associated proteins that function as BMP antagonists (Yamashita *et al.* 1996). BMPs signal via a receptor complex consisting of BMPR-I and BMPR-II. It has been reported that superfamily members TGFβ and TGFβR (Agarwal *et al.* 1997; Lambert *et al.* 1997) and GDNF and GDNFR (Wordinger *et al.* 1999; Liu *et al.* 1999) are expressed by both HTM and ONH cells.

BMPs and BMP receptors are expressed in ocular tissues (Obata *et al.* 1999; You *et al.* 1999), but previous reports have focused on ocular development. The function of BMPs is important in ocular development since targeted disruption of genes encoding BMPs in mice leads to severe developmental defects in the retina and the lens (Jena *et al.* 1997; Luo *et al.* 1995; Dudley *et al.* 1995). BMP-2, BMP-4 and BMP-7 are involved in the development of the lens and retina (Jena *et al.* 1997; Furuta and Hogan 1998; Reddi 2000; Trousse *et al.* 2001). BMP-6 and BMP-7 also appear to play a role in protecting neurons from hypoglycemic or ischemic damage (Nonner *et al.* 2001; Liu *et al.* 2001), and BMP2 has been shown to enhance ganglion cell neurotrophin expression (Zhang *et al.* 1998). Heterozygous knock-out mice haploinsufficient for Bmp4 have ocular phenotypes including anterior segment dysgenesis, elevated IOP, and optic nerve abnormalities (Chang *et al.* 2001). There has been very limited information published concerning the role of BMPs in the human postnatal eye.

Mohan and colleagues (1998) reported that BMP-2 and BMP-4 and BMP receptors were expressed in cells of the adult cornea and suggested that BMP function might include corneal keratocyte proliferation and apoptosis. You and colleagues (1999) verified this study and also reported the expression of BMP-3, BMP-5, and BMP-7 in ex vivo and cultured corneal epithelium and stromal cells. They reported that the level of BMP transcription was higher in the stroma while the level for receptors was higher in cultured corneal epithelial cells.

Using RT-PCR, the present inventors discovered mRNAs for BMPs, BMP receptors BMPR-IA, BMPR-IB and BMPR-II, as well as BMP binding proteins gremlin, chordin, follistatin, and bambi, in HTM, lamina cribosa (LC) and ONH astrocyte cell lines and tissues (Wordinger *et al.* 2002). The present inventors further discovered that HTM and ONH cells express proteins BMP-2, BMP-4, BMP-5 and BMP-7.

Glaucoma will be diagnosed by characterization of genetic changes in genes of members of the BMP signaling family. As used herein, the phrases "bone morphogenic protein family member gene" and "BMP signaling family" refer to all BMPs, BMP receptors and associated proteins. The term "genetic changes" is well known by those skilled in the art. There are numerous examples of diseases associated with genetic changes in specific genes (for examples see Cummings 1997; Strachan, *et al.* 1996; Jorde, *et al.* 1999). Genetic changes in a specific gene (e.g. BMP) can be determined using a variety of techniques well known by those skilled in the art, such as: SSCP, DGGE, ASO, RFLP, heteroduplex analysis, CCM, PTT, and RNase cleavage (see Birren, *et al.* 1998).

Glaucoma may be caused by altered expression of one or more BMP family genes in the eye, which leads to elevated IOP and/or glaucomatous optic neuropathy. "Altered BMP gene expression" means expression of this gene product that is different from normal. The term may also refer to alterations in the sequence of the gene or protein. The normal BMP gene has been well characterized (see above), and the expression of BMP has been reported in a variety of tissues including the TM and ONH. Genetic changes in the coding region of BMP family genes may alter the function of these proteins. Genetic changes outside the coding region may also lead to glaucoma.

It is well known by those skilled in the art that "changes outside" of the coding region of a specific gene are important in the regulation of gene expression. For example, the region upstream (5') of the coding region of most genes is known as the promoter region which "promotes" and regulates the expression of that gene. The promoter region contains numerous nucleotide sequences recognized by various transcription factors and DNA binding proteins that are responsible for activation or repression of gene expression. Regions downstream (3') of the gene can determine poly-adenylation of the gene product, thereby regulating RNA processing and translation of the gene product.

The altered expression of BMP genes or mutations in the sequence of the genes that is indicative of glaucoma may be detected using techniques well known to those of skill in the art. For example, it is contemplated that a nucleic acid fragment of almost any length may be employed, with the total length preferably being limited by the ease of preparation and use in the intended protocol. The nucleic acid sequences disclosed herein may also have utility as probes or primers in nucleic acid hybridization embodiments. As such, it is contemplated that nucleic acid segments that comprise a sequence region that consists of at least a 14 nucleotide long contiguous sequence that has the same sequence as, or is complementary to, a 14 nucleotide long contiguous sequence of BMP-2A (SEQ ID NO:1), BMP-4 (SEQ ID NO:3), BMP-5 (SEQ ID NO:5), BMP-7 (SEQ ID NO:7), BMP-RIA (SEQ ID NO:37), BMP-RIB (SEQ ID NO:39), BMP-RII (SEQ ID NO:41), chordin (SEQ ID NO:43), gremlin (SEQ ID NO:45), follistatin (SEQ ID NO:47), or bambi (SEQ ID NO:53) will find particular utility. Longer contiguous identical or complementary sequences, e.g., those of about 20, 30, 40, 50, 100, 200, 500, 1000 nucleotides (including all intermediate lengths), and even up to full length sequences of about 1547 nucleotides (for BMP-2A), 1946 nucleotides (for BMP-4), 2153 nucleotides (for BMP-5) and 1878 nucleotides (for BMP-7), 2932 nucleotides (for BMP-RIA), 2032 nucleotides (for BMP-RIB), 3611 nucleotides (for BMP-RII), 3561 nucleotides (for chordin), 4049 nucleotides (for gremlin), 1386 nucleotides (for follistatin), and 1523 nucleotides (for bambi) will also be of use in certain embodiments.

It will be readily understood that "intermediate lengths", in this context, means any length between the quoted ranges, such as 14, 15, 16, 17, 18, 19, 20, etc.; 21, 22, 23, etc.; 30, 31, 32, etc.; 50, 51, 52, 53, etc.; 100, 101, 102, 103, etc.; 150, 151, 152, 153, etc.; including all integers through the 200-500; 500-1,000; 1,000-2,000 ranges, up to and including sequences of 2,001, 2002, 2050, 2051, and the like.

The ability of such nucleic acid probes and primers to specifically hybridize to BMP coding sequences and primers to specifically amplify BMP sequences will enable them to be of use in detecting the presence of complementary sequences in a given sample. However, other uses are envisioned, including the use of the sequence information for the preparation of mutant species primers, or primers for use in preparing other genetic constructions.

Nucleic acid molecules having sequence regions consisting of contiguous nucleotide stretches of 10, 20, 30, 50, or even of 100-200 nucleotides or so, identical or complementary to BMP-2A (SEQ ID NO:1), BMP4 (SEQ ID NO:3), BMP-5 (SEQ ID NO:5), BMP7 (SEQ ID NO:7), BMP-RIA (SEQ ID NO:37), BMP-RIB (SEQ ID NO:39), BMP-RII (SEQ ID NO:41), chordin (SEQ ID NO:43), gremlin (SEQ ID NO:45), follistatin (SEQ ID NO:47), or bambi (SEQ ID NO:53) are particularly contemplated as hybridization probes for use in, e.g., SNP evaluation and solid phase hybridization assays, in addition to Southern and northern blotting. This would allow BMP structural or regulatory genes to be analyzed, both in tissues and cells. The total size of fragment, as well as the size of the complementary stretch(es), will ultimately depend on the intended use of application of the particular nucleic acid segment. Smaller fragments will generally find use in hybridization embodiments, wherein the length of the contiguous complementary region may be varied, such as between about 10 and about 100 nucleotides, but larger contiguous complementary stretches of up to about 1547 nucleotides (for BMP-2A), 1946 nucleotides (for BMP-4), 2153 nucleotides (for BMP-5) and 1878 nucleotides (for BMP-7), 2932 nucleotides (for BMP-RIA), 2032 nucleotides (for BMP-RIB), 3611 nucleotides (for BMP-RII), 3561 nucleotides (for chordin), 4049 nucleotides (for gremlin), 1386 nucleotides (for follistatin), and 1523 nucleotides (for bambi) may be used, according to the length complementary sequences one wishes to detect.

The use of a hybridization probe of about 10-14 nucleotides in length allows the formation of a duplex molecule that is both stable and selective. Molecules having contiguous complementary sequences over stretches greater than 10 bases in length are generally preferred, though, in order to increase stability and selectivity of the hybrid, and thereby improve the quality and degree of specific hybrid molecules obtained, one will generally prefer to design nucleic acid molecules having gene-complementary stretches of 15 to 20 contiguous nucleotides, or even longer where desired.

Hybridization probes may be selected from any portion of any of the sequences disclosed herein. All that is required is to review the sequence set forth in SEQ ID NO:1, SEQ ID NO:3, SEQ ID NO:5, SEQ ID NO:7, SEQ ID NO:37, SEQ ID NO:39, SEQ ID NO:41, SEQ ID NO:43, SEQ ID NO:45, SEQ ID NO:47 or SEQ ID NO:53 and to select any continuous portion of the sequence, from about 10 nucleotides in length up to and including the full length sequence, that one wishes to utilize as a probe or primer. The choice of probe and primer sequences may be governed by various factors, such as, by way of example only, one may wish to employ primers from towards the termini of the total sequence, or from the ends of the functional domain-encoding sequences, in order to amplify further DNA.

The process of selecting, and preparing a nucleic acid segment that includes a contiguous sequence from within BMP-2A (SEQ ID NO:1), BMP4 (SEQ ID NO:3), BMP-5 (SEQ ID NO:5), BMP7 (SEQ ID NO:7), BMP-RIA (SEQ ID NO:37), BMP-RIB (SEQ ID NO:39), BMP-RII (SEQ ID NO:41), chordin (SEQ ID NO:43), gremlin (SEQ ID NO:45), follistatin (SEQ ID NO:47), or bambi (SEQ ID NO:53) may alternatively be described as preparing a nucleic acid fragment. Of course, fragments may also be obtained by other techniques such as, e.g., by mechanical shearing or by restriction enzyme digestion. Small nucleic acid segments or fragments may be readily prepared by, for example, directly synthesizing the fragment by chemical means, as is commonly practiced using an automated oligonucleotide synthesizer. Also, fragments may be obtained by application of nucleic acid reproduction technology, such as the PCR™ technology of U.S. Pat. No. 4,683,202 and U.S. Pat. No. 4,682,195 (each incorporated herein by reference), by introducing selected sequences into recombinant vectors for recombinant production, and by other recombinant DNA techniques generally known to those of skill in the art of molecular biology.

Accordingly, the nucleotide sequences of the invention may be used for their ability to selectively form duplex molecules with complementary stretches of BMP genes or cDNAs. Depending on the application envisioned, one will desire to employ varying degrees of selectivity of hybridization to achieve varying degrees of selectivity of probe towards target sequence. For applications requiring high selectivity, one will typically desire to employ relatively stringent conditions to form the hybrids, e.g., one will select relatively low salt and/or high temperature conditions, such as provided by 0.02M-0.15M NaCl at temperatures of 50° C to 70° C. Such selective conditions tolerate little, if any, mismatch between the probe and the template or target strand, and would be particularly suitable for examining BMP genes.

Of course, for some applications, for example, where one desires to prepare or identify mutants employing a mutant primer strand hybridized to an underlying template or where one seeks to isolate BMP encoding sequences from related species, functional equivalents, or the like, less stringent hybridization conditions will typically be needed in order to allow formation of the heteroduplex. In these circumstances, one may desire to employ conditions such a 0.15M-1.0M salt, at temperatures ranging from 20° C to 55° C. Cross-hybridizing species can thereby be readily identified as positively hybridizing signals with respect to control hycbridizations. In any case, it is generally appreciated that conditions can be rendered more stringent by decreasing NaCl concentrations or by the addition of increasing amounts of formamide, which serves to destabilize the hybrid duplex in the same manner as increased temperature. Thus, hybridization conditions can be readily manipulated, and thus will generally be a method of choice depending on the desired results.

In certain embodiments, it will be advantageous to employ nucleic acid sequences of the present invention in combination with an appropriate means, such as a label, for determining hybridization. A wide variety of appropriate indicator means are known in the art, including fluorescent, radioactive, enzymatic or other ligands, such as avidin/biotin, which are capable of giving a detectable signal. In preferred embodiments, one will likely desire to employ a fluorescent label or an enzyme tag, such as urease, alkaline phosphatase or peroxidase, instead of radioactive or other environmental undesirable reagents. In the case of enzyme tags, colorimetric indicator substrates are known that can be employed to provide a means visible to the human eye or spectrophotometrically, to identify specific hybridization with complementary nucleic acid-containing samples.

In general, it is envisioned that the hybridization probes described herein will be useful both as reagents in solution hybridization as well as in embodiments employing a solid phase. In embodiments involving a solid phase, the test DNA (or RNA) is adsorbed or otherwise affixed to a selected matrix or surface. This fixed, single-stranded nucleic acid is then subjected to specific hybridization with selected probes under desired conditions. The selected conditions will depend on the particular circumstances based on the particular criteria required (depending, for example, on the G+C contents, type of target nucleic acid, source of nucleic acid, size of hybridization probe, etc.). Following washing of the hybridized surface so as to remove nonspecifically bound probe molecules, specific hybridization is detected, or even quantified, by means of the label.

It will also be understood that this invention is not limited to the particular nucleic acid and amino acid sequences of BMP-2A (SEQ ID NO:1), BMP4 (SEQ ID NO:3), BMP-5 (SEQ ID NO:5), BMP7 (SEQ ID NO:7), BMP-RIA (SEQ ID NO:37), BMP-RIB (SEQ ID NO:39), BMP-RII (SEQ ID NO:41), chordin (SEQ ID NO:43), gremlin (SEQ ID NO:45), follistatin (SEQ ID NO:47), or bambi (SEQ ID NO:53). Recombinant vectors and isolated DNA segments may therefore variously include the BMP coding regions themselves, upstream or downstream regions of the genes, coding regions bearing selected alterations or modifications in the basic coding region, or they may encode larger polypeptides that nevertheless include BMP coding regions or may encode biologically functional equivalent proteins or polypeptides that have variant amino acid sequences.

The DNA segments of the present invention encompass biologically functional equivalent BMP proteins and polypeptides. Such sequences may arise as a consequence of codon redundancy and functional equivalency that are known to occur naturally within nucleic acid sequences and the proteins thus encoded. Alternatively, functionally equivalent proteins or polypeptides may be created via the application of recombinant DNA technology, in which changes in the protein structure may be engineered, based on considerations of the properties of the amino acids being exchanged. Changes designed by man may be introduced through the application of site-directed mutagenesis techniques, e.g., to introduce improvements to the antigenicity of the protein or to test BMP mutants in order to examine binding activity at the molecular level.

The therapeutic agent for the treatment of glaucoma can be: a peptide or protein, a peptide mimetic, an oligonucleotide or derivatized oligonucleotide, or small drug-like molecule, all which affect one or more aspects of the ocular BMP pathways. Preferred therapeutic agents are those that are: (1) BMP2, BMP4, BMP5, or BMP7 agonists; (2) chordin, gremlin, follistatin, or bambi antagonists; and/or (3) Smad1, Smad5 and/or Smad4 agonists.

The agent may be delivered directly to the eye (for example: topical ocular drops or ointments; slow release devices in the cul-de-sac or implanted adjacent to the sclera or within the eye; periocular, conjunctival, sub-Tenons, intracameral or intravitreal injections) or parenterally (for example: orally; intravenous, subcutaneous or intramuscular injections; dermal delivery; etc.) using techniques well known by those skilled in the art. The following are examples of possible formulations embodied by this invention.

| (a) | Topical ocular formulation | wt. % |
|---|---|---|
| | Agent that increases ocular BMP-4 expression | 0.01 - 2 |
| | HPMC | 0.5 |
| | Sodium chloride | 0.8 |
| | BAC | 0.01% |
| | EDTA | 0.01 |
| | NaOH/HCl | qs pH 7.4 |
| | Purified water | qs 100 mL |

| (b) | Topical ocular formulation | wt. % |
|---|---|---|
| | Gremlin antagonist | 0.01 - 2 |
| | HPMC | 0.5 |
| | Sodium chloride | 0.8 |
| | BAC | 0.01 |
| | EDTA | 0.01 |
| | NaOH/HCl | qs pH 7.4 |
| | Purified water | qs 100 mL |

| (c) | Topical ocular formulation | wt. % |
|---|---|---|
| | Smad 1/5 agonist | 0.01 - 2 |
| | HPMC | 0.5 |
| | Sodium chloride | 0.8 |
| | BAC | 0.01 |
| | EDTA | 0.01 |
| | NaOH/HCl | qs pH 7.2 |
| | Purified water | qs 100 mL |

It is further contemplated that the compounds of the invention could be formulated in intraocular insert devices.

### A. Assay for therapeutic agents

This invention is also useful for the discovery of new anti-glaucoma therapeutic agents that are involved in the BMP signaling pathway (see FIG. 5). Selective BMP ligands bind to BMP type I and type II serine/threonine kinase receptors (BMP-RI and BMP-RII) and transduce signal via Smad proteins. The BMP signal is propagated by Smads through protein-protein and protein-DNA interactions (Attisano and Tuen Lee-Hoeflich 2001). Regulatory Smad 1 and Smad 5 are activated (via phosphorylation) by ligand bound BMP receptors (von Bubnoff and Cho 2001). These regulatory Smads then interact with Smad 4 to form a heteromeric complex that translocates to the nucleus. This complex is able to activate or repress the transcription of selective genes that recognize this transcriptional complex, depending on which nuclear co-factors are present.

The BMP/Smad.signaling pathway is negatively regulated by several mechanisms. Certain BMP-binding proteins (such as gremlin, BAMBI, or follistatin) bind BMPs and inhibit their interaction with BMP receptors. In addition, there are inhibitory Smad proteins (e.g. Smad 6 and Smad 7), which bind and inactivate BMP receptors. (Kowabata *et al.* 1998; Itoh *et al.* 2000; Miyazono 2000). The present inventors have discovered that human TM cells, ONH astrocytes and lamina cribrosa cells express message and protein for the BMP receptor complex. Thus, these cells could respond to endogenous BMP ligands.

Various methods may be used to discover new anti-glaucoma therapeutic agents, and these techniques are well known to those skilled in the art. For example, peptide or peptide mimetic agents that act as agonists or inhibitors of BMPs can be discovered through molecular modeling of BMP/BMP receptor structures (Nickel *et al.* 2001). BMP signal transduction involves select sets of Smad proteins (Kawabata *et al.* 1998; Itoh *et al.* 2000; Attiseno *et al.* 2000). Select BMP agonists and Smad agonists can be discovered using cell based assays. The test cell should express the appropriate BMP receptor(s) and possess the appropriate BMP signaling pathway. Because one of the major effects of BMP signaling is the alteration of gene expression, BMP agonists and Smad agonists can be discovered by screening for BMP-induced genes. The induction of BMP regulated genes also may be assayed by quantitating levels of mRNA using quantitative RT-PCR (Wang *et al.* 2001), DNA microarrays, or reporter gene constructs. There are natural inhibitors of BMP signaling, the BMP binding proteins (also known as BMP-associated proteins), such as chordin, gremlin, and follistatin. Antagonists of the protein inhibitors can be discovered using ligand binding assays. For example, test agents can be added to recombinant purified gremlin, and those agents that bind to gremlin are identified using a variety of techniques known to those skilled in the art. To determine whether these agents are gremlin antagonists, a cell based assay similar to that described above is used.

It is contemplated that any known *in vitro* and *in vivo* screening models may be used in conjunction with the present invention to identify new glaucoma therapies directed to the BMP family of genes. Such models are well known to those skilled in the art and their practice has become routine. Small peptides or peptide mimetics can be designed based on structure/function knowledge of the BMP, BMPR, and/or BMP binding protein gene products. Ligand binding assays can be used to detect small molecules that bind to BMPs, BMPRs, or BMP binding proteins. Cell based assays can look at the effects of various agents on BMP signaling pathways. Knock-in cell lines containing BMP family gene promoters coupled to a reporter gene can be generated to look for agents that alter BMP family member gene expression. These assays can be used to identify both agonist and antagonist molecules. *Ex vivo* assays, such as perfusion cultured anterior segments from human eyes (Clark *et al.* 1995a; Pang *et al.* 2000), can be used to examine the effects of agents on IOP and on BMP signaling in TM tissue. Rodent models of glaucoma can be generated using well-known techniques to create stable BMP family member transgenic, knockout, or knock-in strains of mice and rats. These rodent models can be used to screen for agents that alter the glaucoma-like phenotype(s) (e.g. tonometry to evaluate effects on IOP, histology to evaluate effects on glaucomatous optic neurology).

### B. Kits

The present invention provides methods, compositions and kits for the early detection of glaucoma. The kits can contain a nucleic acid segment encoding a BMP polypeptide or protein. The kit can further contain reagents for detecting an interaction between a sample and a nucleic acid or peptide of the present invention. The provided reagent can be radio-, fluorescently- or enzymatically-labeled. The kit can contain a known radiolabeled agent capable of binding or interacting with a nucleic acid or peptide or protein of the present invention.

The reagent of the kit can be provided as a liquid solution, attached to a solid support or as a dried powder. Preferably, when the reagent is provided in a liquid solution, the liquid solution is an aqueous solution. Preferably, when the reagent provided is attached to a solid support, the solid support can be chromatography media, a test plate having a plurality of wells, or a microscope slide. When the reagent provided is a dry powder, the powder can be reconstituted by the addition of a suitable solvent, that may be provided.

In still further embodiments, the present invention concerns diagnostic methods and associated kits for the diagnosis of glaucoma. It is proposed that the BMP associated peptides and nucleic acids of the invention may be employed to detect polymorphisms or mutations in the BMP nucleic acids from patient samples. In general, these methods will include first obtaining a sample suspected of containing such a polymorphism or mutation, contacting the sample with a peptide or nucleic acid of the present invention, as the case may be, under conditions effective to allow the formation of a complex, and then detecting the presence of the complex.

In general, the detection of complex formation is quite well known in the art and may be achieved through the application of numerous approaches. For example, the present invention contemplates the application of ELISA, RIA, indirect fluorescence techniques and the like. Generally, complex formation will be detected through the use of a label, such as a radiolabel or an enzyme tag (such as alkaline phosphatase, horseradish peroxidase, or the like). Of course, one may find additional advantages through the use of a secondary binding ligand.

The following examples are representative of the techniques employed by the inventors in carrying out aspects of the present invention. It should be appreciated that while these techniques are exemplary of preferred embodiments for the practice of the invention, those of skill in the art, in light of the present disclosure, will recognize that numerous modifications can be made without departing from the spirit and intended scope of the invention.

### Example 1

*Cell culture:* Human TM cells and ONH cells were generated from donor eyes as described (Steely *et al.* 1992; Steely *et al.* 2000; Wilson *et al.* 1993; *Clark et al.* 1994; *Clark et al.* 1995b; *Clark et al.* 1995c; *Clark et al.* 1996; *Clark et al.* 2001 a; Clark *et al.* 2001b; Dickerson *et al.* 1998; Wordinger *et al.* 1998; Wordinger *et al.* 1999; Wordinger *et al.* 2000; Wordinger *et al.* 2002; Lambert *et al.* 2001; Agarwal *et al.* 1999; Liu *et al.* 2001). TM cells were grown from TM explants of donors ranging in age from 6 days to 90 years. Human optic nerve head astrocytes and lamina cribrosa (LC) cells were generated from carefully dissected optic nerve heads (donors aged 2 days to 90 years) and characterized according to previous reports (Lambert *et al.* 2001; *Clark et al.* 1995a). The cells wers grown to confluency in the following media: Ham's F10 media (JRH Biosciences, Lenexa, KS) containing 10% fetal bovine serum (HyClone, Logan, UT) and antibiotics (Gibco BRL-Life Technologies, Grand Island, NY) for TM cells; Dulbecco's modified Eagle's media (DMEM, HyClone) containing 10% FBS for LC cells; and astrocyte growth medium (AGM, Clonetics, San Diego, CA) containing 5% FBS for ONH astrocytes.

*RT-PCR:* Human TM and ONH tissues also were dissected from donor eyes (Wordinger *et al.* 1998; Wang *et al.* 2001). Total RNA was extracted from the TM and ONH cells and tissues using TRIzol extraction (Gibco BRL-Life Technologies), and cDNA prepared by reverse transcription using standard procedures (Wordinger *et al.* 1998; Wordinger *et al.* 1999; Wordinger *et al.* 2000; Wordinger *et al.* 2002). PCR primers were designed using the Oligos 4.0 software program (see primer pairs in Table 1). All primer pairs were designed so that amplification of potentially contaminated genomic DNA sequences would produce mRNA PCR products that would be substantially larger than expected, because intron sequences that were excised during RNA processing would be included in genomic DNA. The β-actin PCR primers, AGGCCAACCGCGAGAAGATGACC (upstream) and GAAGTCCAGGGCGACGTAGCAC (downstream) with an annealing temperature of 55 °C yielded a PCR product of 350 bp.

PCR reactions were run as described (Wordinger *et al.* 1998; Wordinger *et al.* 1999; Wordinger *et al.* 2000; Lambert *et al.* 2001; Wordinger *et al.* 2002) using Taq Start Antibody Hot Start with the following cycle conditions: 2 minutes at 94°C, 2 minutes at 92°C, and 40 cycles of 30 seconds at the optimal annealing temperature, extension for 90 seconds at 72°C and denaturation for 45 seconds at 92°C. The amplified PCR products were examined by horizontal electrophoresis in 1.5% agarose gels. To ensure specificity of the RT-PCR products, Southern blot analysis was performed with probes designed using Oligo 4.0 that hybridized to a region within the amplified PCR product. PCR products were sequenced to verify the specificity of the PCR reactions. Table 2 lists the members of the BMP family that are expressed in the human TM and ONH.

**Table 1. PCR Primer Pairs, Annealing Temperature and Amplimer Size of BMPs**

| Name | Assession Number | Upstream PCR Primer | Downstream PCR Primer | Ampl. Size (bp) |
|---|---|---|---|---|
| BMP-2A | NM_001200 | ACTGCGGTCTCCTAAA GGTCGA (SEQ ID NO:9) | GCTGACCTGAGTGCCT GCGAT (SEQ ID NO: 10) | 657 |
| BMP-4 | NM_001202 | GAATGCTGATGGTCGT TTTTATTATG (SEQ ID NO:11) | AGACTGAAGCCGGTA AAGAT (SEQ ID NO: 12) | 348 |
| BMP-5 | NM_021073 | AAGAGGACAAGAAGG ACTAAAAATAT (SEQ ID NO: 13) | GTAGAGATCCAGCATA AAGAGAGGT (SEQ ID NO:14) | 303 |
| BMP-7 | NM_001719 | AGCCCGGGTAGCGCGT AGAG (SEQ ID NO: 15) | GCGCCGGTGGATGAA GCTCGA (SEQ ID NO:16) | 202 |
| BMPR-1A | NM_004329 | TAAAGGTGACAGTACA CAGGAACA (SEQ ID NO:17) | TCTATGATGGCAAAGC AATGTCC (SEQ ID NO:18) | 298 |
| BMPR-1B | NM_001203 | TACAAGCCTGCCATAA GTGAAGAAGC (SEQ ID NO:19) | ATCATCGTGAAACAAT ATCCGTCTG (SEQ ID NO:20) | 211 |
| BMPR-II | NM_001204 | TCCTCTCATCAGCCAT TTGTCCTTTC (SEQ ID NO:21) | AGTTACTACACATTCT TCATAG (SEQ ID NO:22) | 457 |
| Chordin (CHRD) | AF209930 | CTCTGCTCACTCTGCA CCTG (SEQ ID NO:23) | CCGGTCACCATCAAAA TAGC (SEQ ID NO:24) | 198 |
| Gremlin (CKTSF1 B1) | NM_013372 | ATCAACCGCTTCTGTT ACGG (SEQ ID NO:25) | ATGCAACGACACTGCT TCAC (SEQ ID NO:26) | 197 |
| Follistatin (FST) | NM_006350 | TGCCACCTGAGAAAGG CTAC (SEQ ID NO:27) | ACAGACAGGCTCATCC GACT (SEQ ID NO:28) | 201 |
| Noggin (NOG) | NM_005450 | CACTACGACCCAGGCT TCAT (SEQ ID NO:29) | CTCCGCAGCTTCTTGC TTAG (SEQ ID NO:30) | 212 |
| CER-1 | NM_005454 | ATAGTGAGCCCTTCCC ACCT (SEQ ID NO:33) | AATGAACAGACCCGC ATTTC (SEQ ID NO:34) | 294 |
| NMA (BAMBI) | NM_005791 | GATCGCCACTCCAGCT ACATC (SEQ ID NO:35) | GGGCACGGCAATGAC C (SEQ ID NO:36) | 471 |

**Table 2. BMP Family Members Expressed in Human TM and ONH**

| BMP Family Member | Trabecular Meshwork | Optic Nerve Head |
|---|---|---|
| BMP-2 | + | + |
| BMP-4 | + | + |
| BMP-5 | + | + |
| BMP-7 | + | + |
| BMPR-IA | + | + |
| BMPR-IB | + | + |
| BMPR-II | + | + |
| Chordin | + | + |
| Gremlin | + | + |
| Follistatin | + | + |
| Bambi | + | + |
| Noggin | - | - |
| CER-1 | - | - |

*Western immunoblotting:* Protein was extracted from cultured cells using lysis buffer, and proteins were separated by denaturing polyacrylamide gel electrophoresis prior to electrophoretic transfer to nitrocellulose membranes (Lambert *et al.* 2001). The membranes were blocked with 5% milk (for BMPs) or 3% gelatin (for BMPRs) and incubated with the following primary antibodies: BMP2, BMP4, BMP5, BMP7 (all from Santa Cruz, Santa Cruz, CA), or BMP-RIA, BMP-RIB, BMP-RII (from Jackson Immuno Research, West Grove, PA). The membranes were washed, incubated with secondary antibodies (goat anti-mouse IgG-horseradish peroxidase for BMPs, Santa Cruz; donkey anti-goat-horseradish peroxidase for BMP receptors, Jackson Immuno Research), and developed using the WesternBreeze chemiluminescence immunodetection system (Invitrogen, Carlsbad, CA).

*Expression of BMPs, BMPRs mRNA in human TM cells and tissues:* Amplification products of expected for BMP-2, BMP-4, BMP-5 and BMP-7 primer pairs in human TM cells and tissues are shown in FIG. 6. Southern blots using specific probes verified that these were the expected PCR products. All human TM cell lines and tissues expressed message for BMP-2, BMP-4, and BMP-7. However, message for BMP-5 was low to undetectable in human TM tissue samples (FIG. 6, lanes 6 and 7). Control reactions without cDNA did not result in amplification products indicating that reagents and primers were free of DNA or RNA contamination (FIG. 6, lane C).

FIG. 7 shows the amplification products of expected size for BMP-RIA, BMP-RIB, and BMP-RII primer pairs in human TM cells and tissues. All human TM cells and tissues expressed message for the BMP receptor complexes. Southern blots using specific probes verified that these were the expected PCR products. An alternate amplification product (350 bp) was detected in the BMP-RII PCR reaction. The alternate amplification product was present in all human TM cells and tissues. This alternate band is currently being identified to determine if it is an alternate spliced form of the receptor. Control reactions without cDNA did not result in amplification products (FIG. 7, lane C) indicating that reagents and primers were free of DNA or RNA contamination.

### Expression of BMP and BMP receptor mRNA in human ONH cells and tissues:

Amplification products of expected size for BMP-2, BMP-4, BMP-5 and BMP-7 primer pairs in human ONH astrocytes and ONH tissues are shown in FIG. 8. All ONH astrocytes and ONH tissue expressed message for the respective BMP. Human brain astrocytes were used as a positive control cell line. Southern blots using specific probes verified that these were the expected PCR products. With the exception of BMP-2, all other BMP were expressed by human brain astrocytes (FIG. 8, lane 7). Control reactions without cDNA did not result in amplification products (FIG. 8, lane C) indicating that reagents and primers were free of DNA or RNA contamination.

FIG. 9 shows the amplification products of expected sizes for BMP-2, BMP-4, BMP-5 and BMP-7 primer pairs in cultured human LC cells. All LC cell lines expressed message for each BMP. Southern blots using specific probes verified that these were the expected PCR products. Control reactions without cDNA did not result in amplification products (FIG. 9, lane C) indicating that reagents and primers were free of DNA or RNA contamination.

Amplification products of expected size for BMP-RIA, BMP-RIB, and BMP-RII primer pairs in human ONH astrocytes and ONH tissues are shown in FIG. 10. All ONH astrocyte cell lines and tissues expressed message for BMP-RIA and BMP-RIB. Southern blots using specific probes verified that these were the expected PCR products. With the exception of ONH tissue (FIG. 10, lane 6), BMP-RII was expressed by all ONH astrocyte cell lines. Message for all BMP receptors (FIG. 10, lane 7) was expressed by a human brain astrocyte cell line that served as a positive control. There appears to be a discrepancy in the expression of BMP-RII in ONH tissue and ONH cell lines. The reduced expression in ONH tissue may reflect a low level of expression. Control reactions without cDNA did not result in amplification products (FIG. 5, lane C) indicating that reagents and primers were free of DNA or RNA contamination.

FIG. 11 shows the amplification products of expected size for BMP-RIA, BMP-RIB, and BMP-RII primer pairs in cultured human LC cells. All LC cell lines expressed message for each BMP receptor. Southern blots using specific probes verified that these were the expected PCR products. Control reactions without cDNA did not result in amplification products (FIG. 11, lane C) indicating that reagents and primers were free of DNA or RNA contamination.

*Expression of BMP proteins and BMP receptor proteins in human TM and ONH cells and tissues:* FIG. 12 represents chemiluminescent immunoblot detection of BMP-2, BMP-4, BMP-5, BMP-7, BMP-RIA, BMP-RIB, and BMP-RII proteins in human TM and ONH cells and tissues. All cell lines studied expressed the respective BMP proteins. The BMP proteins were detected in cell lines the following molecular weights: 54-56 kDa for BMP-2, 25-27 kDa for BMP-4, 55-57 kDa for BMP-5, and 77 kDa for BMP-7. Multiple bands were detected for BMP-2 and BMP-4, which most likely represent glycosylated, and partially glycosylated forms of these BMPs as seen in other studies. However, we did not do glycosylation studies as they were beyond the scope of this study. The BMP receptor proteins were detected in cell lines at molecular weights: 38 kDa for BMP-RIA, 64 kDa for BMP-RIB, and 57 kDa for BMP-RII. Multiple bands were detected for BMP-RIB and BMP-RII in the TM cells, which most likely represent glycosylated, and partially glycosylated forms as seen in other studies. The expression levels of proteins for the BMP receptors appeared to be lower in the TM cells compared to ONH cells. For example BMP-RII was not detected in TM cells and BMP-RIB was greatly reduced.

*Expression of BMP associated protein mRNAs in cultured human TM cells and in human ONH cells:* Amplification products of expected size for BMP associated protein primer pairs in human TM cell lines are shown in FIG. 13. Human TM cell lines expressed message for DRM (gremlin), chordin, follistatin, and NMA (BAMBI). Southern blots using specific probes verified that these were the expected PCR products. There was no apparent difference in message expression between cell lines. All human TM cells examined failed to express mRNA for the BMP associated proteins noggin and Cer-1. Control reactions without cDNA did not result in amplification products indicating that reagents and primers were free of DNA or RNA contamination.

Amplification products of expected size for BMP associated protein primer pairs in ONH astrocytes and LC cell lines are shown in FIG. 14. All ONH astrocytes and LC cell lines expressed message for DRM (gremlin), follistatin and NMA (BAMBI). Southern blots using specific probes verified that these were the expected PCR products. The majority of LC cells and ONH astrocytes expressed message for chordin. All human ONH astrocytes and LC cells examined failed to express mRNA for the BMP associated proteins noggin and Cer-1. Control reactions without cDNA did not result in amplification products indicating that reagents and primers were free of DNA and RNA contamination.

FIG. 15 shows increased expression of the BMP antagonist gremlin (CKTSF1B1) in glaucomatous TM cells. Gene expression was assessed using Affymetrix gene arrays (Affymetrix gene chip U133A).

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and structurally related may be substituted for the agents described herein to achieve similar results. All such substitutions and modifications apparent to those skilled in the art are deemed to be within the spirit, scope and concept of the invention as defined by the appended claims.

### References

The following references, to the extent that they provide exemplary procedural or other details supplementary to those set forth herein, are specifically incorporated herein by reference.

### Books

Birren, et al., GENOME ANALYSIS, Vol. 2, (1998).

Clark AF, Browder S, Steely HT, Wilson K, Cantu-Crouch D, McCartney MD, "Cell biology of the human lamina cribrosa," In Drance SM (ed). OPTIC NERVE IN GLAUCOMA. Kugler Publications, New York: pp. 79-105 (1995b).

Cummings, Michael R., HUMAN HEREDITY, Fourth Edition, (1997).

Grierson I, Calthorpe CM, "Characteristics of meshwork cells and age changes in the outflow system of the eye: their relevance to primary open angle glaucoma." In Mills KB (ed). GLAUCOMA. PROCEEDINGS OF THE FOURTH INTERNATIONAL SYMPOSIUM OF THE NORTHERN EYE INSTITUTE, Manchester, UK, New York, Pergamon: pp. 12-31 (1988).

Hernandez M, Gong H, "Extracellular matrix of the trabecular meshwork and optic nerve head." in Ritch R., Shields, M.B., Krupin, T. (eds). THE GLAUCOMAS, 2nd ed. St Louis: Mosby-Year; pp. 213-249 (1996).

Jorde, et al., MEDICAL GENETICS, Second Edition, (1999).

Lutjen-Drecoll E., Rohen J.W., "Morphology of aqueous outflow pathways in normal and glaucomatous eyes," in Ritch R., Shields, M.B., Krupin, T. (eds). THE GLAUCOMAS, 2nd ed. St Louis: Mosby-Year; pp. 89-123 (1996).

Strachan, et al., HUMAN MOLECULAR GENETICS, (1996).

Tripathi RC, Borisuth NS, Li, J, Tripathi BJ, "Clinical implications of aqueous humor growth factors in glaucoma," in Ritch R., Shields, M.B., Krupin, T. (eds). THE GLAUCOMAS, 2nd ed. St Louis: Mosby-Year; pp. 71-87 (1996).

Varma R, Minckler D, "Anatomy and pathophysiology of the retina and optic nerve." in Ritch R., Shields, M.B., Krupin, T. (eds). THE GLAUCOMAS, 2nd ed. St Louis: Mosby-Year; pp. 139-175 (1996).

Vaughan, D. et al., In: GENERAL OPHTHALMOLOGY, Appleton & Lange, Norwalk, Conn., pp. 213-230 (1992).

### Other Publications

Agarwal et al., IOVS 38(4):S563 (1997)

Agarwal R, Talati M, Lambert W, Clark AF, Wilson SE, Agarwal N, Wordinger RJ, "FAS-activated apoptosis and other apoptosis mediators in human trabecular meshwork cells," Exp. Eye Res. 68:583-590 (1999).

Astrom, A.K., Jin, D., Imamura, T., Roijer, E., Rosenzweig, B., Miyazono, K., ten Dijke, P., Stenman, G., Mamm. Genome 10(3):299-302 (1999).

Attisano L, Tuen Lee-Hoeflich S, "The Smads," Genome Biol. 2:REVIEWS3010 (2001).

Bengtsson, B., Br. J. Ophthalmol. 73:483-487 (1989).

Chang B, Smith RS, Peters M, Savinova DV, Hawes NL, Zabalata A, Nusinowitz S, Martin JE, Davisson ML, Sepko CL, Hogan BML, John SWM, "Haploinsufficient Bmp4 ocular phenotypes include anterior segment dysgenesis with elevated intraocular pressure," BMC Genetics 2:18 (2001).

Chundru RK, Agarwal R, Wordinger RJ, Whitson JT, "Detection of neurotrophins in human aqueous humor," Invest. Ophthalmol. Vis. Sci. 41:S236 (2000).

Clark AF, Kawase K, English-Wright S, Lane D, Steely HT, Yamamoto T, Kitazawa Y, Kwon YH, Fingert JH, Swiderski RE, Mullins RF, Hageman GS, Alward WLM, Sheffield VC, Stone EM, "Expression of the glaucoma gene myocilin (MYOC) in the human optic nerve head," FASEB J. 15:1251-1253 (2001).

Clark AF, Lane D, Wilson K, Miggans ST, McCartney MD, "Inhibition of dexamethasone-induced cytoskeletal changes in cultured human trabecular meshwork cells by tetrahydrocortisol," Invest. Ophthalmol. Vis. Sci. 35:805-813 (1996).

Clark AF, Miggans ST, Wilson K, Browder S, McCartney MD, "Cytoskeletal changes in cultured human glaucoma trabecular meshwork cells," J. Glaucoma 4:183-188 (1995c).

Clark AF, Steely HT, Dickerson JE, English-Wright S, Stropki K, McCartney MD, Jacobson N, Shepard AR, Clark JI, Matsushima H, Peskind ER, Leverenz JB, Wilkinson CW, Swiderski RE, Fingert JH, Sheffield VC, Stone EM, "Glucocorticoid induction of the glaucoma gene MYOC in human and monkey trabecular meshwork cells and tissues," Invest. Ophthalmol. Vis. Sci. 42:1769-1780 (2001b).

Clark AF, Wilson K, de Kater AW, Allingham RR, McCartney MD, "Dexamethasone-induced ocular hypertension in perfusion-cultured human eyes," Invest. Ophthalmol. Vis. Sci. 36:478-489 (1995a).

Clark AF, Wilson K, McCartney MD, Miggans ST, Kunkle M, Howe W, "Glucocorticoid-induced formation of cross-linked actin networks in cultured human trabecular meshwork cells," Invest. Ophthalmol. Vis. Sci. 35:281-294 (1994).

Dickerson JE, Steely HT, English-Wright SL, Clark AF, "The effect of dexamethasone on integrin and laminin expression in cultured human trabecular meshwork cells," Exp. Eye Res. 66:731-738 (1998).

Dudley AT, Lyons KM, Robertson EJ, "A requirement for bone morphogenic protein-7 during development of the mammalian kidney and eye," Genes Dev. 9:2795-2807 (1995).

Furuta Y, Hogan BL, "BMP4 is essential for lens induction in the mouse embryo," Genes Dev. 12:3764-3775 (1998).

Greve, M. et al., Can. J. Ophthamol. 28:201-206 (1993).

Giguère et al., Cell 46:645-652 (1986).

Hernandez MR, Andrzejewska WM, Neufeld AH, "Changes in the extracellular matrix of the human optic nerve head in primary open-angle glaucoma," Am. J. Ophthalmol. 109:180-188 (1990).

Hernandez MR, Pena JD, "The optic nerve head in glaucomatous optic neuropathy," Arch Ophthalmol. 115:389-395 (1997).

Hitchings, R. A., Br. J. Ophthamol. 77:326 (1993).

Hogan BL, "Bone morphogenic proteins: multifunctional regulators of vertebrate development," Genes Dev. 10:1580-1594 (1996).

Hu DN, Ritch R, "Hepatocyte growth factor is increased in the aqueous humor of glaucomatous eyes," J. Glaucoma 10:152-157 (2001).

Inatani M, Tanihara H, Katsuta H, Honjo M, Kido N, Honda Y, "Transforming growth factor beta 2 levels in aqueous humor of glaucomatous eyes," Graefes Arch. Clin. Exp. Ophthalmol. 239:109-113 (2001).

Itoh et al., Eur. J. Biochem. 267:6954-6967 (2000).

Jena N, Martin-Seisdedos C, McCue P, Croce CM, "BMP7 null mutation in mice: developmental defects in skeleton, kidney, and eye," Exp. Cell Res. 230:28-37 (1997).

Kawabata et al., Cytokine & Growth Factor Review, 9:49-61 (1998).

Kerrigan LA, Zack DJ, Quigley HA, Smith SD, Pease ME, "TUNEL-positive ganglion cells in human primary open-angle glaucoma," Arch. Ophthalmol. 115:1031-1035 (1997).

Lambert et al., IOVS 38(4):S162 (1997).

Lambert W, Agarwal R, Howe W, Clark AF, Wordinger RJ, "Neurotrophin and neurotrophin receptor expression by cells of the human lamina cribrosa," Invest. Ophthalmol. Vis. Sci., 42:2315-2323 (2001).

Leske, M. C. et al., Amer. J. Epidemiol. 113:1843-1846 (1986).

Liu et al., IOVS 40(4):S673 (1999).

Liu Y, Belayev L, Zhao W, Busto R, Saul I, Alonso O, Ginsberg MD, "The effect of bone morphogenic protein-7 (BMP-7) on functional recovery, local cerebral glucose utilization and blood flow after transient focal cerebral ischemia in rats," Brain Res. 905:81-90 (2001).

Liu X, Lambert W, Agarwal R, Talati M, Cross W, Clark AF, Wordinger RJ, "Human trabecular meshwork cells express the ciliary neurotrophic factor (CNTF) tripartate receptor complex," Exp. Eye Res. 72:711-717 (2001).

Luo G, Gofmann C, Bronckers AL, Sohocki M, Bradley A, Karsenty G, "BMP-7 is an inducer of nephrogenesis, and is also required for eye development and skeletal patterning," Genes Dev. 9:2808-2820 (1995).

McMahon, R., Murphy, M., Clarkson, M., Taal, M., Mackenzie, H.S., Godson, C., Martin, F., Brady, H.R., J. Biol. Chem. 275(14):9901-9904 (2000).

Miyazono, J. Cell Science, 113:1101-1109 (2000).

Mohan RR, Kim WJ, Mohan RR, Chen L, Wilson SE, "Bone morphogenic proteins 2 and 4 and their receptors in the adults human cornea," Invest. Ophthalmol. Vis. Sci. 39:2626-2636 (1998).

Morrison JC, Dorman-Pease ME, Dunkelberger GR, Quigley HA, "Optic nerve head extracellular matrix in primary optic atrophy and experimental glaucoma," Arch. Ophthalmol. 108:1020-1024 (1990).

Murphy, M., Godson, C., Cannon, S., Kato, S., Mackenzie, H.S., Martin, F., Brady, H.R., J. Biol. Chem. 274(9):5830-5834 (1999).

Nickel J, Dreyer MK, Kirsch T, Sebold W, "The crystal structure of BMP-2:BMPR-1A complex and the generation of BMP-2 antagonists," J. Bone & Joint Surgery 83-A(suppl 1):S1-S7 (2001).

Nohno, T., Ishikawa, T., Saito, T., Hosokawa, K., Noji, S., Wolsing, D.H., Rosenbaum, J.S., J. Biol. Chem. 270(38):22522-22526 (1995).

Nonner D, Barrett EF, Kaplan P, Barrett JN, "Bone morphogenic proteins (BMP6 and BMP7) enhance the protective effect of neurotrophins on cultured septal cholinergic neurons during hypoglycemia," J. Neurochem. 77:691-699 (2001).

Obata H, Kaji Y, Yamada H, Kato M, Tsuru T, Yamashita H, "Expression of tranfsorming growth factor-beta superfamily receptors in rat eyes," Acta. Ophthalmol. Scand. 77:151-156 (1999).

Pang I-H, McCartney MD, Steely HT, Clark AF, "Human ocular perfusion organ culture: a versatile ex vivo model for glaucoma research," J. Glaucoma 9:468-479 (2000).

Pena JD, Taylor AW, Ricard CS, Vidal I, Hemandez MR, "Transforming growth factor beta isoforms in human optic nerve heads," Br. J. Ophthalmol. 83:209-218 (1999).

Picht G, Welge-Luessen U, Grehn F, Lutjen-Drecoll E, "Transforming growth factor beta 2 levels in the aqueous humor in different types of glaucoma and the relation to filtering bleb development," Graefes Arch. Clin. Exp. Ophthalmol. 239:199-207 (2001).

Quigley HA, McKinnon SJ, Zack DJ, Pease ME< Kerrigan-Baumrind LA, Kerrigan DF, Mitchell RS, "Retrograde axonal transport of BDNF in retinal ganglion cells is blocked by acute IOP elevation in rats," Invest. Ophthalmol. Vis. Sci. 41:3460-3466 (2000).

Quigley HA, "Neuronal death in glaucoma," Prog. Retin. Eye Res. 18:39-57 (1999).

Quigley HA, Nickells RW, Kerrigan LA, Pease ME, Thibault DJ, Zack DJ, "Retinal ganglion cell death in experimental glaucoma and after axotomy occurs by apoptosis," Invest. Ophthalmol. Vis. Sci. 36:774-786 (1995).

Reddi AH, "Bone morphonegetic proteins: an unconventional approach to isolation of first mammalian morphogens," Cytokine Growth Factor Rev. 8:11-20 (1997).

Reddi AH, "Bone morphogenic proteins and skeletal development: the kidney-bone connection," Pediatr. Nephrol. 14:598-601 (2000).

Rohen JW, "Why is intraocular pressure elevated in chronic simple glaucoma? Anatomical considerations." Ophthalmology 90:758-765 (1983).

Steely HT, Browder SL, Julian MB, Miggans ST, Wilson KL, Clark AF, "The effects of dexamethasone on fibronectin expression in cultured human trabecular meshwork cells," Invest. Ophthalmol. Vis. Sci. 33: 2242-2250 (1992).

Steely HT, English-Wright SL, Clark AF, "Similarity of protein expression in trabecular meshwork and lamina cribrosa: implications for glaucoma," Exp. Eye Res. 70:17-30 (2000).

Strong, N. P., Ophthal. Physiol. Opt. 12:3-7 (1992).

ten Dijke, P.P., Ichijo, H., Franzen, P., Schulz, P., Saras, J., Toyoshima, H., Heldin, C.H., Miyazono, K., Oncogene 8(10):2879-2887 (1993).

Tripathi RC, Borisuth NS, Kolli SP, Tripathi BJ, "Trabecular cells express receptors that bind TGF-beta 1 and TGF-beta 2: a qualitative and quantitative characterization," Invest. Ophthalmol. Vis. Sci. 34:260-263 (1993b).

Tripathi RC, Borisuth NS, Tripathi BJ, "Detection, quantification, and significance of basic fibroblast growth factor in the aqueous humor of man, cat, dog and pig," Exp. Eye Res. 54:447-454 (1992).

Tripathi RC, Borisuth NS, Tripathi BJ, Fang VS, "Analysis of human aqueous humor for epidermal growth factor," Exp. Eye Res. 53:407-409 (1991).

Tripathi RC, Chan WF, Li J, Tripathi BJ, "Trabecular cells express the TFG-beta 2 gene and secrete the cytokine," Exp. Eye Res. 58:523-528 (1994a).

Tripathi RC, Li J, Borisuth NS, Tripathi BJ, "Trabecular cells of the eye express messenger RNA for transforming growth factor-beta 1 and secrete this cytokine," Invest. Ophthalmol. Vis. Sci. 34:2562-2569 (1993a).

Tripathi RC, Li J, Chan WF, Tripathi BJ, "Aqueous humor in glaucomatous eyes contains an increased level of TFG-beta 2," Exp. Eye Res. 59:723-727 (1994c).

Tripathi RC, Li J, Tripathi BJ, "Immunolocalization of bFGF in the trabecular meshwork and detection of its mRNA in trabecular cells," Exp. Eye Res. 58:503-507 (1994b).

Trousse F, Esteve P, Bovolenta P, "BMP4 mediates apoptotic cell death in the developing chick eye," J. Neurosci. 21:1292-1301 (2001).

Tuck, M. W. et al., Ophthal. Physiol. Opt. 13:227-232 (1993).

Vernon, S. A., Eye 7:134-137 (1993).

Von Bubnoff A, Cho KW, "Intracellular BMP signaling regulation in vertebrates: pathway or network?" Dev. Biol. 239:1-14 (2001).

Wang W-H, McNatt LG, Shepard AR, Jacobson N, Nishimura DY, Stone EM, Sheffield VC, Clark AF, "Optimal procedure for extracting RNA from human ocular tissues and expression profiling of the congenital glaucoma gene FOXC1 using quantitative RT-PCR," Molecular Vision 7:89-94 (2001).

Wilson K, McCartney MD, Miggans ST, Clark AF, "Dexamethasone induced ultrastructural changes in cultured human trabecular meshwork cells," Current Eye Research 12:783-793 (1993).

Wordinger et al., IOVS 40(4):S504 (1999a).

Wordinger RJ, Agarwal R, Talati M, Fuller J, Lambert W, Calrk AF, "Expression of bone morphogenic proteins (BMP), BMP receptors, and BMP associated proteins in human trabecular meshwork and optic nerve head cells and tissues," Molec. Vision 8:241-256 (2002).

Wordinger RJ, Clark AF, Agarwal R, Lambert W, McNatt L, Wilson SE, Qu E, Fung BK-K, "Cultured human trabecular meshwork cells express functional growth factor receptors," Invest. Ophthalmol. Vis. Sci. 39: 1575-1589 (1998).

Wordinger RJ, Clark AF, Agarwal R, Lambert W, Wilson SE, "Expression of alternatively spliced growth factor receptor isoforms in the human trabecular meshwork," Invest. Ophthalmol. Vis. Sci. 40:242-247 (1999b).

Wordinger RJ, Lambert W, Agarwal R, Talati M, Clark AF, "Human trabecular meshwork cells secrete neurotrophins and express neurotrophin receptors (Trk)," Invest. Ophthalmol. Vis. Sci. 41:3833-3841 (2000).

Yamashita H, Ten Dijke P, Heldin CH, Miyazono K, "Bone morphogenic protein receptors," Bone 19:569-574 (1996).

You L, Kruse FE, Pohl J, Volcker HE, "Bone morphogenic proteins and growth and differentiation factors in the human cornea," Invest. Ophthalmol. Vis. Sci. 40:296-311 (1999).

Zhang D, Mehler MF, Song Q, Kessler JA, "Development of bone morphogenic protein receptors in the nervous system and possible roles in regulating trkC expression," J. Neurosci. 18:3314-3326 (1998).

SEQUENCE LISTING

<110> Alcon Research, Ltd.
Clark, Abbot F.

<120> Bone Morphogenic Proteins (BMP), BMP Receptors and BMP Binding Proteins and Their U
se in the Diagnosis and Treatment of Glaucoma

<130> 2312 US

<160> 54

<170> PatentIn version 3.1

<210> 1
<211> 1547
<212> DNA
<213> homo sapiens

<400> SEQ ID NO:1
ggggacttct tgaacttgca gggagaataa cttgcgcacc ccactttgcg ccggtgcctt 60

tgccccagcg gagcctgctt cgccatctcc gagccccacc gcccctccac tcctcggcct 120

tgcccgacac tgagacgctg ttcccagcgt gaaaagagag actgcgcggc cggcacccgg 180

gagaaggagg aggcaaagaa aaggaacgga cattcggtcc ttgcgccagg tcctttgacc 240

agagtttttc catgtggacg ctctttcaat ggacgtgtcc ccgcgtgctt cttagacgga 300

ctgcggtctc ctaaaggtcg accatggtgg ccgggacccg ctgtcttcta gcgttgctgc 360

ttccccaggt cctcctgggc ggcgcggctg gcctcgttcc ggagctgggc cgcaggaagt 420

tcgcggcggc gtcgtcgggc cgcccctcat cccagccctc tgacgaggtc ctgagcgagt 480

tcgagttgcg gctgctcagc atgttcggcc tgaaacagag acccaccccc agcagggacg 540

ccgtggtgcc cccctacatg ctagacctgt atcgcaggca ctcaggtcag ccgggctcac 600

ccgccccaga ccaccggttg gagagggcag ccagccgagc caacactgtg cgcagcttcc 660

accatgaaga atctttggaa gaactaccag aaacgagtgg gaaaacaacc cggagattct 720

tctttaattt aagttctatc cccacggagg agtttatcac ctcagcagag cttcaggttt 780

tccgagaaca gatgcaagat gctttaggaa acaatagcag tttccatcac cgaattaata 840

tttatgaaat cataaaacct gcaacagcca actcgaaatt ccccgtgacc agacttttgg 900

acaccaggtt ggtgaatcag aatgcaagca ggtgggaaag ttttgatgtc acccccgctg 960

tgatgcggtg gactgcacag ggacacgcca accatggatt cgtggtggaa gtggcccact 1020

tggaggagaa acaaggtgtc tccaagagac atgttaggat aagcaggtct ttgcaccaag 1080

atgaacacag ctggtcacag ataaggccat tgctagtaac ttttggccat gatggaaaag 1140

ggcatcctct ccacaaaaga gaaaaacgtc aagccaaaca caaacagcgg aaacgcctta 1200

agtccagctg taagagacac cctttgtacg tggacttcag tgacgtgggg tggaatgact 1260

ggattgtggc tcccccgggg tatcacgcct tttactgcca cggagaatgc ccttttcctc 1320

tggctgatca tctgaactcc actaatcatg ccattgttca gacgttggtc aactctgtta 1380

actctaagat tcctaaggca tgctgtgtcc cgacagaact cagtgctatc tcgatgctgt 1440

accttgacga gaatgaaaag gttgtattaa agaactatca ggacatggtt gtggagggtt 1500

gtgggtgtcg ctagtacagc aaaattaaat acataaatat atatata                    1547

<210>  2
<211>  396
<212>  PRT
<213>  homo sapiens

<400>  SEQ ID NO:2

Met Val Ala Gly Thr Arg Cys Leu Leu Ala Leu Leu Leu Pro Gln Val
1               5                   10                  15

Leu Leu Gly Gly Ala Ala Gly Leu Val Pro Glu Leu Gly Arg Arg Lys
            20                  25                  30

Phe Ala Ala Ala Ser Ser Gly Arg Pro Ser Ser Gln Pro Ser Asp Glu
        35                  40                  45

Val Leu Ser Glu Phe Glu Leu Arg Leu Leu Ser Met Phe Gly Leu Lys
    50                  55                  60

Gln Arg Pro Thr Pro Ser Arg Asp Ala Val Val Pro Pro Tyr Met Leu
65                  70                  75                  80

Asp Leu Tyr Arg Arg His Ser Gly Gln Pro Gly Ser Pro Ala Pro Asp
                85                  90                  95

His Arg Leu Glu Arg Ala Ala Ser Arg Ala Asn Thr Val Arg Ser Phe
            100                 105                 110

His His Glu Glu Ser Leu Glu Glu Leu Pro Glu Thr Ser Gly Lys Thr
        115                 120                 125

Thr Arg Arg Phe Phe Phe Asn Leu Ser Ser Ile Pro Thr Glu Glu Phe
    130                 135                 140

Ile Thr Ser Ala Glu Leu Gln Val Phe Arg Glu Gln Met Gln Asp Ala
145                 150                 155                 160

Leu Gly Asn Asn Ser Ser Phe His His Arg Ile Asn Ile Tyr Glu Ile
                165                 170                 175

Ile Lys Pro Ala Thr Ala Asn Ser Lys Phe Pro Val Thr Arg Leu Leu
            180                 185                 190

Asp Thr Arg Leu Val Asn Gln Asn Ala Ser Arg Trp Glu Ser Phe Asp
        195                 200                 205

Val Thr Pro Ala Val Met Arg Trp Thr Ala Gln Gly His Ala Asn His
    210                 215                 220

Gly Phe Val Val Glu Val Ala His Leu Glu Glu Lys Gln Gly Val Ser
225                 230                 235                 240

Lys Arg His Val Arg Ile Ser Arg Ser Leu His Gln Asp Glu His Ser
                245                 250                 255

Trp Ser Gln Ile Arg Pro Leu Leu Val Thr Phe Gly His Asp Gly Lys
            260                 265                 270

Gly His Pro Leu His Lys Arg Glu Lys Arg Gln Ala Lys His Lys Gln
        275                 280                 285

Arg Lys Arg Leu Lys Ser Ser Cys Lys Arg His Pro Leu Tyr Val Asp
    290                 295                 300

Phe Ser Asp Val Gly Trp Asn Asp Trp Ile Val Ala Pro Pro Gly Tyr
305                 310                 315                 320

His Ala Phe Tyr Cys His Gly Glu Cys Pro Phe Pro Leu Ala Asp His
                325                 330                 335

Leu Asn Ser Thr Asn His Ala Ile Val Gln Thr Leu Val Asn Ser Val
            340                 345                 350

Asn Ser Lys Ile Pro Lys Ala Cys Cys Val Pro Thr Glu Leu Ser Ala
        355                 360                 365

Ile Ser Met Leu Tyr Leu Asp Glu Asn Glu Lys Val Val Leu Lys Asn
    370                 375                 380

Tyr Gln Asp Met Val Val Glu Gly Cys Gly Cys Arg
385                 390                 395

<210>  3
<211>  1946
<212>  DNA
<213>  homo sapiens

<400>  SEQ ID NO:3
gaaagcgagg gagggaaaga ggaggaagga agatgcgaga aggcagagga ggagggaggg      60

agggaaggag cgcggagccc ggcccggaag ctaggtgagt gtggcatccg agctgaggga     120

cgcgagcctg agacgccgct gctgctccgg ctgagtatct agcttgtctc cccgatggga     180

ttcccgtcca agctatctcg agcctgcagc gccacagtcc ccggccctcg cccaggttca     240

ctgcaaccgt tcagaggtcc ccaggagctg ctgctggcga gcccgctact gcagggacct     300

atggagccat tccgtagtgc catcccgagc aacgcactgc tgcagcttcc ctgagccttt     360

ccagcaagtt tgttcaagat tggctgtcaa gaatcatgga ctgttattat atgccttgtt     420

ttctgtcaag acaccatgat tcctggtaac cgaatgctga tggtcgtttt attatgccaa     480

gtcctgctag gaggcgcgag ccatgctagt ttgatacctg agacggggaa gaaaaaagtc     540

gccgagattc agggccacgc gggaggacgc cgctcagggc agagccatga gctcctgcgg     600

gacttcgagg cgacacttct gcagatgttt gggctgcgcc gccgcccgca gcctagcaag     660

agtgccgtca ttccggacta catgcgggat ctttaccggc ttcagtctgg ggaggaggag     720

gaagagcaga tccacagcac tggtcttgag tatcctgagc gcccggccag ccgggccaac 780

accgtgagga gcttccacca cgaagaacat ctggagaaca tcccagggac cagtgaaaac 840

tctgcttttc gtttcctctt taacctcagc agcatccctg agaacgaggc gatctcctct 900

gcagagcttc ggctcttccg ggagcaggtg gaccagggcc ctgattggga aaggggcttc 960

caccgtataa acatttatga ggttatgaag cccccagcag aagtggtgcc tgggcacctc 1020

atcacacgac tactggacac gagactggtc caccacaatg tgacacggtg ggaaactttt 1080

gatgtgagcc ctgcggtcct tcgctggacc cgggagaagc agccaaacta tgggctagcc 1140

attgaggtga ctcacctcca tcagactcgg acccaccagg gccagcatgt caggattagc 1200

cgatcgttac ctcaagggag tgggaattgg gcccagctcc ggcccctcct ggtcaccttt 1260

ggccatgatg gccggggcca tgccttgacc cgacgccgga gggccaagcg tagccctaag 1320

catcactcac agcgggccag gaagaagaat aagaactgcc ggcgccactc gctctatgtg 1380

gacttcagcg atgtgggctg gaatgactgg attgtggccc caccaggcta ccaggccttc 1440

tactgccatg gggactgccc ctttccactg gctgaccacc tcaactcaac caaccatgcc 1500

attgtgcaga ccctggtcaa ttctgtcaat tccagtatcc ccaaagcctg ttgtgtgccc 1560

actgaactga gtgccatctc catgctgtac ctggatgagt atgataaggt ggtactgaaa 1620

aattatcagg agatggtagt agagggatgt gggtgccgct gagatcaggc agtccttgag 1680

gatagacaga tatacacacc acacacacac accacataca ccacacacac acgttcccat 1740

ccactcaccc acacactaca cagactgctt ccttatagct ggacttttat ttaaaaaaaa 1800

aaaaaaaaaa atggaaaaaa tccctaaaca ttcaccttga ccttatttat gactttacgt 1860

gcaaatgttt tgaccatatt gatcatatat tttgacaaaa tatatttata actacgtatt 1920

aaaagaaaaa aataaaatga gtcatt 1946

<210> 4
<211> 408
<212> PRT
<213> homo sapiens

<400> SEQ ID NO:4

Met Ile Pro Gly Asn Arg Met Leu Met Val Val Leu Leu Cys Gln Val
1               5                   10                  15

Leu Leu Gly Gly Ala Ser His Ala Ser Leu Ile Pro Glu Thr Gly Lys
            20                  25                  30

Lys Lys Val Ala Glu Ile Gln Gly His Ala Gly Gly Arg Arg Ser Gly
        35                  40                  45

Gln Ser His Glu Leu Leu Arg Asp Phe Glu Ala Thr Leu Leu Gln Met
    50                  55                  60

Phe Gly Leu Arg Arg Arg Pro Gln Pro Ser Lys Ser Ala Val Ile Pro
65                  70                  75                  80

Asp Tyr Met Arg Asp Leu Tyr Arg Leu Gln Ser Gly Glu Glu Glu
                85                  90                  95

Glu Gln Ile His Ser Thr Gly Leu Glu Tyr Pro Glu Arg Pro Ala Ser
            100                 105                 110

Arg Ala Asn Thr Val Arg Ser Phe His His Glu Glu His Leu Glu Asn
        115                 120                 125

Ile Pro Gly Thr Ser Glu Asn Ser Ala Phe Arg Phe Leu Phe Asn Leu
    130                 135                 140

Ser Ser Ile Pro Glu Asn Glu Ala Ile Ser Ser Ala Glu Leu Arg Leu
145                 150                 155                 160

Phe Arg Glu Gln Val Asp Gln Gly Pro Asp Trp Glu Arg Gly Phe His
                165                 170                 175

Arg Ile Asn Ile Tyr Glu Val Met Lys Pro Pro Ala Glu Val Val Pro
            180                 185                 190

Gly His Leu Ile Thr Arg Leu Leu Asp Thr Arg Leu Val His His Asn
        195                 200                 205

Val Thr Arg Trp Glu Thr Phe Asp Val Ser Pro Ala Val Leu Arg Trp
    210                 215                 220

Thr Arg Glu Lys Gln Pro Asn Tyr Gly Leu Ala Ile Glu Val Thr His
225                 230                 235                 240

Leu His Gln Thr Arg Thr His Gln Gly Gln His Val Arg Ile Ser Arg
                245                 250                 255

Ser Leu Pro Gln Gly Ser Gly Asn Trp Ala Gln Leu Arg Pro Leu Leu
            260                 265                 270

Val Thr Phe Gly His Asp Gly Arg Gly His Ala Leu Thr Arg Arg Arg
        275                 280                 285

Arg Ala Lys Arg Ser Pro Lys His His Ser Gln Arg Ala Arg Lys Lys
    290                 295                 300

Asn Lys Asn Cys Arg Arg His Ser Leu Tyr Val Asp Phe Ser Asp Val
305                 310                 315                 320

Gly Trp Asn Asp Trp Ile Val Ala Pro Pro Gly Tyr Gln Ala Phe Tyr
                325                 330                 335

Cys His Gly Asp Cys Pro Phe Pro Leu Ala Asp His Leu Asn Ser Thr
            340                 345                 350

Asn His Ala Ile Val Gln Thr Leu Val Asn Ser Val Asn Ser Ser Ile
        355                 360                 365

Pro Lys Ala Cys Cys Val Pro Thr Glu Leu Ser Ala Ile Ser Met Leu
    370                 375                 380

Tyr Leu Asp Glu Tyr Asp Lys Val Val Leu Lys Asn Tyr Gln Glu Met
385                 390                 395                 400

Val Val Glu Gly Cys Gly Cys Arg
                405

<210>  5
<211>  2153
<212>  DNA
<213>  homo sapiens

<400>  SEQ ID NO:5
ctggtatatt tgtgcctgct ggaggtggaa ttaacagtaa gaaggagaaa gggattgaat      60

ggacttacag gaaggatttc aagtaaattc agggaaacac atttacttga atagtacaac     120

ctagagtatt attttacact aagacgacac aaaagatgtt aaagttatca ccaagctgcc     180

ggacagatat atattccaac accaaggtgc agatcagcat agatctgtga ttcagaaatc     240

aggatttgtt ttggaaagag ctcaagggtt gagaagaact caaaagcaag tgaagattac     300

tttgggaact acagtttatc agaagatcaa cttttgctaa ttcaaatacc aaaggcctga     360

ttatcataaa ttcatatagg aatgcatagg tcatctgatc aaataatatt agccgtcttc     420

tgctacatca atgcagcaaa aactcttaac aactgtggat aattggaaat ctgagtttca     480

gctttcttag aaataactac tcttgacata ttccaaaata tttaaaatag gacaggaaaa     540

tcggtgagga tgttgtgctc agaaatgtca ctgtcatgaa aaataggtaa atttgttttt     600

tcagctactg ggaaactgta cctcctagaa ccttaggttt tttttttttt aagaggacaa     660

gaaggactaa aaatatcaac ttttgctttt ggacaaaaat gcatctgact gtatttttac     720

ttaagggtat tgtgggtttc ctctggagct gctgggttct agtgggttat gcaaaaggag     780

gtttgggaga caatcatgtt cactccagtt ttatttatag aagactacgg aaccacgaaa     840

gacgggaaat acaaagggaa attctctcta tcttgggttt gcctcacaga cccagaccat     900

tttcacctgg aaaacaagcg tcctctgcac ctctctttat gctggatctc tacaatgcca     960

tgaccaatga agaaaatcct gaagagtcgg agtactcagt aagggcatcc ttggcagaag    1020

agaccagagg ggcaagaaag ggatacccag cctctcccaa tgggtatcct cgtcgcatac    1080

agttatctcg gacgactcct ctgaccaccc agagtcctcc tctagccagc ctccatgata    1140

ccaactttct gaatgatgct gacatggtca tgagctttgt caacttagtt gaaagagaca    1200

aggatttttc tcaccagcga aggcattaca aagaatttcg atttgatctt acccaaattc    1260

ctcatggaga ggcagtgaca gcagctgaat tccggatata caaggaccgg agcaacaacc    1320

gatttgaaaa tgaaacaatt aagattagca tatatcaaat catcaaggaa tacacaaata    1380

gggatgcaga tctgttcttg ttagacacaa gaaaggccca agctttagat gtgggttggc    1440

ttgtctttga tatcactgtg accagcaatc attgggtgat taatccccag aataatttgg    1500

gcttacagct ctgtgcagaa acaggggatg gacgcagtat caacgtaaaa tctgctggtc 1560

ttgtgggaag acagggacct cagtcaaaac aaccattcat ggtggccttc ttcaaggcga 1620

gtgaggtact tcttcgatcc gtgagagcag ccaacaaacg aaaaaatcaa aaccgcaata 1680

aatccagctc tcatcaggac tcctccagaa tgtccagtgt tggagattat aacacaagtg 1740

agcaaaaaca agcctgtaag aagcacgaac tctatgtgag cttccgggat ctgggatggc 1800

aggactggat tatagcacca gaaggatacg ctgcatttta ttgtgatgga gaatgttctt 1860

ttccacttaa cgcccatatg aatgccacca accacgctat agttcagact ctggttcatc 1920

tgatgtttcc tgaccacgta ccaaagcctt gttgtgctcc aaccaaatta aatgccatct 1980

ctgttctgta ctttgatgac agctccaatg tcattttgaa aaaatataga aatatggtag 2040

tacgctcatg tggctgccac taatattaaa taatattgat aataacaaaa agatctgtat 2100

taaggtttat ggctgcaata aaaagcatac tttcagacaa acagaaaaaa aaa 2153

<210> 6
<211> 454
<212> PRT
<213> homo sapiens

<400> SEQ ID NO:6

Met His Leu Thr Val Phe Leu Leu Lys Gly Ile Val Gly Phe Leu Trp
1               5                   10                  15

Ser Cys Trp Val Leu Val Gly Tyr Ala Lys Gly Gly Leu Gly Asp Asn
            20                  25                  30

His Val His Ser Ser Phe Ile Tyr Arg Arg Leu Arg Asn His Glu Arg
        35                  40                  45

Arg Glu Ile Gln Arg Glu Ile Leu Ser Ile Leu Gly Leu Pro His Arg
    50                  55                  60

Pro Arg Pro Phe Ser Pro Gly Lys Gln Ala Ser Ser Ala Pro Leu Phe
65                  70                  75                  80

Met Leu Asp Leu Tyr Asn Ala Met Thr Asn Glu Glu Asn Pro Glu Glu
                85                  90                  95

Ser Glu Tyr Ser Val Arg Ala Ser Leu Ala Glu Glu Thr Arg Gly Ala
            100                 105                 110

Arg Lys Gly Tyr Pro Ala Ser Pro Asn Gly Tyr Pro Arg Arg Ile Gln
        115                 120                 125

Leu Ser Arg Thr Thr Pro Leu Thr Thr Gln Ser Pro Pro Leu Ala Ser
    130                 135                 140

Leu His Asp Thr Asn Phe Leu Asn Asp Ala Asp Met Val Met Ser Phe
145                 150                 155                 160

Val Asn Leu Val Glu Arg Asp Lys Asp Phe Ser His Gln Arg Arg His
                165                 170                 175

Tyr Lys Glu Phe Arg Phe Asp Leu Thr Gln Ile Pro His Gly Glu Ala
            180                 185                 190

Val Thr Ala Ala Glu Phe Arg Ile Tyr Lys Asp Arg Ser Asn Asn Arg
        195                 200                 205

Phe Glu Asn Glu Thr Ile Lys Ile Ser Ile Tyr Gln Ile Ile Lys Glu
    210                 215                 220

Tyr Thr Asn Arg Asp Ala Asp Leu Phe Leu Leu Asp Thr Arg Lys Ala
225                 230                 235                 240

Gln Ala Leu Asp Val Gly Trp Leu Val Phe Asp Ile Thr Val Thr Ser
                245                 250                 255

Asn His Trp Val Ile Asn Pro Gln Asn Asn Leu Gly Leu Gln Leu Cys
            260                 265                 270

Ala Glu Thr Gly Asp Gly Arg Ser Ile Asn Val Lys Ser Ala Gly Leu
        275                 280                 285

Val Gly Arg Gln Gly Pro Gln Ser Lys Gln Pro Phe Met Val Ala Phe
    290                 295                 300

Phe Lys Ala Ser Glu Val Leu Leu Arg Ser Val Arg Ala Ala Asn Lys
305                 310                 315                 320

Arg Lys Asn Gln Asn Arg Asn Lys Ser Ser Ser His Gln Asp Ser Ser
                325                 330                 335

Arg Met Ser Ser Val Gly Asp Tyr Asn Thr Ser Glu Gln Lys Gln Ala
            340                 345                 350

Cys Lys Lys His Glu Leu Tyr Val Ser Phe Arg Asp Leu Gly Trp Gln
        355                 360                 365

Asp Trp Ile Ile Ala Pro Glu Gly Tyr Ala Ala Phe Tyr Cys Asp Gly
    370                 375                 380

Glu Cys Ser Phe Pro Leu Asn Ala His Met Asn Ala Thr Asn His Ala
385                 390                 395                 400

Ile Val Gln Thr Leu Val His Leu Met Phe Pro Asp His Val Pro Lys
                405                 410                 415

Pro Cys Cys Ala Pro Thr Lys Leu Asn Ala Ile Ser Val Leu Tyr Phe
            420                 425                 430

Asp Asp Ser Ser Asn Val Ile Leu Lys Lys Tyr Arg Asn Met Val Val
        435                 440                 445

Arg Ser Cys Gly Cys His
    450

<210>  7
<211>  1878
<212>  DNA
<213>  homo sapiens

<400>  SEQ ID NO:7
gggcgcagcg gggcccgtct gcagcaagtg accgacggcc gggacggccg cctgccccct      60

ctgccacctg ggcggtgcgg ggcccggagc ccggagcccg ggtagcgcgt agagccggcg     120

cgatgcacgt gcgctcactg cgagctgcgg cgccgcacag cttcgtggcg ctctgggcac     180

ccctgttcct gctgcgctcc gccctggccg acttcagcct ggacaacgag gtgcactcga     240

gcttcatcca ccggcgcctc cgcagccagg agcggcggga gatgcagcgc gagatcctct     300

ccatttgggg cttgccccac cgcccgcgcc cgcacctcca gggcaagcac aactcggcac     360

ccatgttcat gctggacctg tacaacgcca tggcggtgga ggagggcggc gggcccggcg     420

gccagggctt ctcctacccc tacaaggccg tcttcagtac ccagggcccc cctctggcca     480

gcctgcaaga tagccatttc ctcaccgacg ccgacatggt catgagcttc gtcaacctcg     540

tggaacatga caaggaattc ttccacccac gctaccacca tcgagagttc cggtttgatc     600

tttccaagat cccagaaggg gaagctgtca cggcagccga attccggatc tacaaggact     660

acatccggga acgcttcgac aatgagacgt tccggatcag cgtttatcag gtgctccagg     720

agcacttggg cagggaatcg gatctcttcc tgctcgacag ccgtaccctc tgggcctcgg     780

aggagggctg gctggtgttt gacatcacag ccaccagcaa ccactgggtg gtcaatccgc     840

ggcacaacct gggcctgcag ctctcggtgg agacgctgga tgggcagagc atcaacccca     900

agttggcggg cctgattggg cggcacgggc cccagaacaa gcagcccttc atggtggctt     960

tcttcaaggc cacggaggtc cacttccgca gcatccggtc cacggggagc aaacagcgca    1020

gccagaaccg ctccaagacg cccaagaacc aggaagccct gcggatggcc aacgtggcag    1080

agaacagcag cagcgaccag aggcaggcct gtaagaagca cgagctgtat gtcagcttcc    1140

gagacctggg ctggcaggac tggatcatcg cgcctgaagg ctacgccgcc tactactgtg    1200

agggggagtg tgccttccct ctgaactcct acatgaacgc caccaaccac gccatcgtgc    1260

agacgctggt ccacttcatc aacccggaaa cggtgcccaa gccctgctgt gcgcccacgc    1320

agctcaatgc catctccgtc ctctacttcg atgacagctc caacgtcatc ctgaagaaat    1380

acagaaacat ggtggtccgg gcctgtggct gccactagct cctccgagaa ttcagaccct    1440

ttggggccaa gtttttctgg atcctccatt gctcgccttg gccaggaacc agcagaccaa    1500

ctgccttttg tgagaccttc ccctccctat ccccaacttt aaaggtgtga gagtattagg    1560

aaacatgagc agcatatggc ttttgatcag tttttcagtg gcagcatcca atgaacaaga    1620

tcctacaagc tgtgcaggca aaacctagca ggaaaaaaaa acaacgcata aagaaaaatg    1680

gccgggccag gtcattggct gggaagtctc agccatgcac ggactcgttt ccagaggtaa    1740

ttatgagcgc ctaccagcca ggccacccag ccgtgggagg aagggggcgt ggcaaggggt 1800

gggcacattg gtgtctgtgc gaaaggaaaa ttgacccgga agttcctgta ataaatgtca 1860

caataaaacg aatgaatg 1878

<210> 8
<211> 431
<212> PRT
<213> homo sapiens

<400> SEQ ID NO:8

Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Gly Gly
                85                  90                  95

Gly Pro Gly Gly Gln Gly Phe Ser Tyr Pro Tyr Lys Ala Val Phe Ser
            100                 105                 110

Thr Gln Gly Pro Pro Leu Ala Ser Leu Gln Asp Ser His Phe Leu Thr
        115                 120                 125

Asp Ala Asp Met Val Met Ser Phe Val Asn Leu Val Glu His Asp Lys
    130                 135                 140

Glu Phe Phe His Pro Arg Tyr His His Arg Glu Phe Arg Phe Asp Leu
145                 150                 155                 160

Ser Lys Ile Pro Glu Gly Glu Ala Val Thr Ala Ala Glu Phe Arg Ile
                165                 170                 175

Tyr Lys Asp Tyr Ile Arg Glu Arg Phe Asp Asn Glu Thr Phe Arg Ile
            180                 185                 190

Ser Val Tyr Gln Val Leu Gln Glu His Leu Gly Arg Glu Ser Asp Leu
        195                 200                 205

Phe Leu Leu Asp Ser Arg Thr Leu Trp Ala Ser Glu Glu Gly Trp Leu
    210                 215                 220

Val Phe Asp Ile Thr Ala Thr Ser Asn His Trp Val Val Asn Pro Arg
225                 230                 235                 240

His Asn Leu Gly Leu Gln Leu Ser Val Glu Thr Leu Asp Gly Gln Ser
                245                 250                 255

Ile Asn Pro Lys Leu Ala Gly Leu Ile Gly Arg His Gly Pro Gln Asn
            260                 265                 270

Lys Gln Pro Phe Met Val Ala Phe Phe Lys Ala Thr Glu Val His Phe
        275                 280                 285

Arg Ser Ile Arg Ser Thr Gly Ser Lys Gln Arg Ser Gln Asn Arg Ser
    290                 295                 300

Lys Thr Pro Lys Asn Gln Glu Ala Leu Arg Met Ala Asn Val Ala Glu
305                 310                 315                 320

Asn Ser Ser Ser Asp Gln Arg Gln Ala Cys Lys Lys His Glu Leu Tyr
                325                 330                 335

Val Ser Phe Arg Asp Leu Gly Trp Gln Asp Trp Ile Ile Ala Pro Glu
            340                 345                 350

Gly Tyr Ala Ala Tyr Tyr Cys Glu Gly Glu Cys Ala Phe Pro Leu Asn
        355                 360                 365

Ser Tyr Met Asn Ala Thr Asn His Ala Ile Val Gln Thr Leu Val His
    370                 375                 380

Phe Ile Asn Pro Glu Thr Val Pro Lys Pro Cys Cys Ala Pro Thr Gln
385                 390                 395                 400

Leu Asn Ala Ile Ser Val Leu Tyr Phe Asp Asp Ser Ser Asn Val Ile
                405                 410                 415

Leu Lys Lys Tyr Arg Asn Met Val Val Arg Ala Cys Gly Cys His
            420                 425                 430

<210>  9
<211>  22
<212>  DNA
<213>  homo sapiens

<400>  SEQ ID NO:9
actgcggtct cctaaaggtc ga                                              22

<210>  10
<211>  21
<212>  DNA
<213>  homo sapiens

<400>  SEQ ID NO:10
gctgacctga gtgcctgcga t                                               21

<210> 11
<211> 26
<212> DNA
<213> homo sapiens

<400> SEQ ID NO:11
gaatgctgat ggtcgttttt attatg 26

<210> 12
<211> 20
<212> DNA
<213> homo sapiens

<400> SEQ ID NO:12
agactgaagc cggtaaagat 20

<210> 13
<211> 26
<212> DNA
<213> homo sapiens

<400> SEQ ID NO:13
aagaggacaa gaaggactaa aaatat 26

<210> 14
<211> 25
<212> DNA
<213> homo sapiens

<400> SEQ ID NO:14
gtagagatcc agcataaaga gaggt 25

<210> 15
<211> 20
<212> DNA
<213> homo sapiens

<400> SEQ ID NO:15
agcccgggta gcgcgtagag 20

<210> 16
<211> 21
<212> DNA
<213> homo sapiens

<400> SEQ ID NO:16
gcgccggtgg atgaagctcg a 21

<210> 17
<211> 24
<212> DNA
<213> homo sapiens

<400> SEQ ID NO:17
taaaggtgac agtacacagg aaca 24

<210> 18
<211> 23
<212> DNA
<213> homo sapiens

<400> SEQ ID NO:18
tctatgatgg caaagcaatg tcc 23

<210> 19
<211> 26
<212> DNA
<213> homo sapiens

<400> SEQ ID NO:19
tacaagcctg ccataagtga agaagc 26

<210> 20
<211> 25
<212> DNA
<213> homo sapiens

<400> SEQ ID NO:20
atcatcgtga aacaatatcc gtctg 25

<210> 21
<211> 26
<212> DNA
<213> homo sapiens

<400> SEQ ID NO:21
tcctctcatc agccatttgt cctttc 26

<210> 22
<211> 22
<212> DNA
<213> homo sapiens

<400> SEQ ID NO:22
agttactaca cattcttcat ag 22

<210> 23
<211> 20
<212> DNA
<213> homo sapiens

<400> SEQ ID NO:23
ctctgctcac tctgcacctg 20

<210> 24
<211> 20
<212> DNA
<213> homo sapiens

<400> SEQ ID NO:24
ccggtcacca tcaaaatagc 20

<210> 25
<211> 20
<212> DNA
<213> homo sapiens

<400> SEQ ID NO:25
atcaaccgct tctgttacgg 20

<210> 26
<211> 20
<212> DNA
<213> homo sapiens

<400> SEQ ID NO:26
atgcaacgac actgcttcac 20

<210> 27
<211> 20
<212> DNA
<213> homo sapiens

<400> SEQ ID NO:27
tgccacctga gaaaggctac 20

<210> 28
<211> 20
<212> DNA
<213> homo sapiens

<400> SEQ ID NO:28
acagacaggc tcatccgact 20

<210> 29
<211> 20
<212> DNA
<213> homo sapiens

<400> SEQ ID NO:29
cactacgacc caggcttcat 20

<210> 30
<211> 20
<212> DNA
<213> homo sapiens

<400> SEQ ID NO:30
ctccgcagct tcttgcttag 20

<210> 31
<211> 20
<212> DNA
<213> homo sapiens

<400> SEQ ID NO:31
atccttcttc atctggctgc 20

<210> 32
<211> 20
<212> DNA
<213> homo sapiens

<400> SEQ ID NO:32
aattggtgtc ctgaggatcg 20

<210> 33
<211> 20
<212> DNA
<213> homo sapiens

<400> SEQ ID NO:33
atagtgagcc cttcccacct 20

<210> 34
<211> 20
<212> DNA

<213>  homo sapiens

<400>  SEQ ID NO:34
aatgaacaga cccgcatttc                                                    20


<210>  35
<211>  21
<212>  DNA
<213>  homo sapiens

<400>  SEQ ID NO:35
gatcgccact ccagctacat c                                                  21


<210>  36
<211>  16
<212>  DNA
<213>  homo sapiens

<400>  SEQ ID NO:36
gggcacggca atgacc                                                        16


<210>  37
<211>  2932
<212>  DNA
<213>  homo sapiens

<400>  SEQ ID NO:37
gctccgcgcc gagggctgga ggatgcgttc cctggggtcc ggacttatga aaatatgcat      60

cagtttaata ctgtcttgga attcatgaga tggaagcata ggtcaaagct gtttggagaa     120

aatcagaagt acagttttat ctagccacat cttggaggag tcgtaagaaa gcagtgggag     180

ttgaagtcat tgtcaagtgc ttgcgatctt ttacaagaaa atctcactga atgatagtca     240

tttaaattgg tgaagtagca agaccaatta ttaaaggtga cagtacacag gaaacattac     300

aattgaacaa tgactcagct atacatttac atcagattat tgggagccta tttgttcatc     360

atttctcgtg ttcaaggaca gaatctggat agtatgcttc atggcactgg gatgaaatca     420

gactccgacc agaaaaagtc agaaaatgga gtaaccttag caccagagga taccttgcct     480

tttttaaagt gctattgctc agggcactgt ccagatgatg ctattaataa cacatgcata     540

actaatggac attgctttgc catcatagaa gaagatgacc agggagaaac cacattagct     600

tcagggtgta tgaaatatga aggatctgat tttcagtgca aagattctcc aaaagcccag     660

ctacgccgga caatagaatg ttgtcggacc aatttatgta accagtattt gcaacccaca     720

ctgccccctg ttgtcatagg tccgtttttt gatggcagca ttcgatggct ggttttgctc     780

atttctatgg ctgtctgcat aattgctatg atcatcttct ccagctgctt ttgttacaaa     840

cattattgca agagcatctc aagcagacgt cgttacaatc gtgatttgga acaggatgaa     900

gcatttattc cagttggaga atcactaaaa gaccttattg accagtcaca aagttctggt     960

agtgggtctg gactaccttt attggttcag cgaactattg ccaaacagat tcagatggtc    1020

cggcaagttg gtaaaggccg atatggagaa gtatggatgg gcaaatggcg tggcgaaaaa    1080

gtggcggtga aagtattctt taccactgaa gaagccagct ggtttcgaga aacagaaatc    1140

taccaaactg tgctaatgcg ccatgaaaac atacttggtt tcatagcggc agacattaaa    1200

ggtacaggtt cctggactca gctctatttg attactgatt accatgaaaa tggatctctc 1260

tatgacttcc tgaaatgtgc tacactggac accagagccc tgcttaaatt ggcttattca 1320

gctgcctgtg gtctgtgcca cctgcacaca gaaatttatg gcacccaagg aaagcccgca 1380

attgctcatc gagacctaaa gagcaaaaac atcctcatca agaaaaatgg gagttgctgc 1440

attgctgacc tgggccttgc tgttaaattc aacagtgaca caaatgaagt tgatgtgccc 1500

ttgaatacca gggtgggcac caaacgctac atggctcccg aagtgctgga cgaaagcctg 1560

aacaaaaacc acttccagcc ctacatcatg gctgacatct acagcttcgg cctaatcatt 1620

tgggagatgg ctcgtcgttg tatcacagga gggatcgtgg aagaatacca attgccatat 1680

tacaacatgg taccgagtga tccgtcatac gaagatatgc gtgaggttgt gtgtgtcaaa 1740

cgtttgcggc caattgtgtc taatcggtgg aacagtgatg aatgtctacg agcagttttg 1800

aagctaatgt cagaatgctg ggcccacaat ccagcctcca gactcacagc attgagaatt 1860

aagaagacgc ttgccaagat ggttgaatcc caagatgtaa aaatctgatg gttaaaccat 1920

cggaggagaa actctagact gcaagaactg tttttaccca tggcatgggt ggaattagag 1980

tggaataagg atgttaactt ggttctcaga ctctttcttc actacgtgtt cacaggctgc 2040

taatattaaa cctttcagta ctcttattag gatacaagct gggaacttct aaacacttca 2100

ttctttatat atggacagct ttattttaaa tgtggttttt gatgcctttt tttaagtggg 2160

tttttatgaa ctgcatcaag acttcaatcc tgattagtgt ctccagtcaa gctctgggta 2220

ctgaattgcc tgttcataaa acggtgcttt ctgtgaaagc cttaagaaga taaatgagcg 2280

cagcagagat ggagaaatag actttgcctt ttacctgaga cattcagttc gtttgtattc 2340

tacctttgta aaacagccta tagatgatga tgtgtttggg atactgctta ttttatgata 2400

gtttgtcctg tgtccttagt gatgtgtgtg tgtctccatg cacatgcacg ccgggattcc 2460

tctgctgcca tttgaattag aagaaaataa tttatatgca tgcacaggaa gatattggtg 2520

gccggtggtt ttgtgcttta aaaatgcaat atctgaccaa gattcgccaa tctcatacaa 2580

gccatttact ttgcaagtga gatagcttcc ccaccagctt tattttttaa catgaaagct 2640

gatgccaagg ccaaaagaag tttaaagcat ctgtaaattt ggactgtttt ccttcaacca 2700

ccattttttt tgtggttatt atttttgtca cggaaagcat cctctccaaa gttggagctt 2760

ctattgccat gaaccatgct tacaaagaaa gcacttctta ttgaagtgaa ttcctgcatt 2820

tgatagcaat gtaagtgcct ataaccatgt tctatattct ttattctcag taacttttaa 2880

aagggaagtt atttatattt tgtgtataat gtgctttatt tgcaaatcac cc 2932

<210> 38
<211> 532
<212> PRT
<213> homo sapiens

<400> SEQ ID NO:38

Met Thr Gln Leu Tyr Ile Tyr Ile Arg Leu Leu Gly Ala Tyr Leu Phe
1               5                   10                  15

Ile Ile Ser Arg Val Gln Gly Gln Asn Leu Asp Ser Met Leu His Gly
            20                  25                  30

Thr Gly Met Lys Ser Asp Ser Asp Gln Lys Lys Ser Glu Asn Gly Val
        35                  40                  45

Thr Leu Ala Pro Glu Asp Thr Leu Pro Phe Leu Lys Cys Tyr Cys Ser
    50                  55                  60

Gly His Cys Pro Asp Asp Ala Ile Asn Asn Thr Cys Ile Thr Asn Gly
65                  70                  75                  80

His Cys Phe Ala Ile Ile Glu Glu Asp Asp Gln Gly Glu Thr Thr Leu
                85                  90                  95

Ala Ser Gly Cys Met Lys Tyr Glu Gly Ser Asp Phe Gln Cys Lys Asp
            100                 105                 110

Ser Pro Lys Ala Gln Leu Arg Arg Thr Ile Glu Cys Cys Arg Thr Asn
        115                 120                 125

Leu Cys Asn Gln Tyr Leu Gln Pro Thr Leu Pro Pro Val Val Ile Gly
    130                 135                 140

Pro Phe Phe Asp Gly Ser Ile Arg Trp Leu Val Leu Leu Ile Ser Met
145                 150                 155                 160

Ala Val Cys Ile Ile Ala Met Ile Ile Phe Ser Ser Cys Phe Cys Tyr
                165                 170                 175

Lys His Tyr Cys Lys Ser Ile Ser Ser Arg Arg Arg Tyr Asn Arg Asp
            180                 185                 190

Leu Glu Gln Asp Glu Ala Phe Ile Pro Val Gly Glu Ser Leu Lys Asp
        195                 200                 205

Leu Ile Asp Gln Ser Gln Ser Ser Gly Ser Gly Ser Gly Leu Pro Leu
    210                 215                 220

Leu Val Gln Arg Thr Ile Ala Lys Gln Ile Gln Met Val Arg Gln Val
225                 230                 235                 240

Gly Lys Gly Arg Tyr Gly Glu Val Trp Met Gly Lys Trp Arg Gly Glu
                245                 250                 255

Lys Val Ala Val Lys Val Phe Phe Thr Thr Glu Glu Ala Ser Trp Phe
            260                 265                 270

Arg Glu Thr Glu Ile Tyr Gln Thr Val Leu Met Arg His Glu Asn Ile
        275                 280                 285

Leu Gly Phe Ile Ala Ala Asp Ile Lys Gly Thr Gly Ser Trp Thr Gln
    290                 295                 300

Leu Tyr Leu Ile Thr Asp Tyr His Glu Asn Gly Ser Leu Tyr Asp Phe
305                 310                 315                 320

Leu Lys Cys Ala Thr Leu Asp Thr Arg Ala Leu Leu Lys Leu Ala Tyr
                325                 330                 335

Ser Ala Ala Cys Gly Leu Cys His Leu His Thr Glu Ile Tyr Gly Thr
            340                 345                 350

Gln Gly Lys Pro Ala Ile Ala His Arg Asp Leu Lys Ser Lys Asn Ile
        355                 360                 365

Leu Ile Lys Lys Asn Gly Ser Cys Cys Ile Ala Asp Leu Gly Leu Ala
    370                 375                 380

Val Lys Phe Asn Ser Asp Thr Asn Glu Val Asp Val Pro Leu Asn Thr
385                 390                 395                 400

Arg Val Gly Thr Lys Arg Tyr Met Ala Pro Glu Val Leu Asp Glu Ser
                405                 410                 415

Leu Asn Lys Asn His Phe Gln Pro Tyr Ile Met Ala Asp Ile Tyr Ser
            420                 425                 430

Phe Gly Leu Ile Ile Trp Glu Met Ala Arg Arg Cys Ile Thr Gly Gly
        435                 440                 445

Ile Val Glu Glu Tyr Gln Leu Pro Tyr Tyr Asn Met Val Pro Ser Asp
    450                 455                 460

Pro Ser Tyr Glu Asp Met Arg Glu Val Val Cys Val Lys Arg Leu Arg
465                 470                 475                 480

Pro Ile Val Ser Asn Arg Trp Asn Ser Asp Glu Cys Leu Arg Ala Val
                485                 490                 495

Leu Lys Leu Met Ser Glu Cys Trp Ala His Asn Pro Ala Ser Arg Leu
            500                 505                 510

Thr Ala Leu Arg Ile Lys Lys Thr Leu Ala Lys Met Val Glu Ser Gln
        515                 520                 525

Asp Val Lys Ile
    530

<210>  39
<211>  2032
<212>  DNA
<213>  homo sapiens

<400>  SEQ ID NO:39
cgcggggcgc ggagtcggcg gggcctcgcg ggacgcgggc agtgcggaga ccgcggcgct      60

gaggacgcgg gagccgggag cgcacgcgcg gggtggagtt cagcctactc tttcttagat 120
gtgaaaggaa aggaagatca tttcatgcct tgttgataaa ggttcagact tctgctgatt 180
cataaccatt tggctctgag ctatgacaag agaggaaaca aaaagttaaa cttacaagcc 240
tgccataagt gagaagcaaa cttccttgat aacatgcttt tgcgaagtgc aggaaaatta 300
aatgtgggca ccaagaaaga ggatggtgag agtacagccc ccaccccccg tccaaaggtc 360
ttgcgttgta aatgccacca ccattgtcca gaagactcag tcaacaatat ttgcagcaca 420
gacggatatt gtttcacgat gatagaagag gatgactctg ggttgcctgt ggtcacttct 480
ggttgcctag gactagaagg ctcagatttt cagtgtcggg acactcccat tcctcatcaa 540
agaagatcaa ttgaatgctg cacagaaagg aacgaatgta ataaagacct acaccctaca 600
ctgcctccat tgaaaaacag agattttgtt gatggaccta tacaccacag ggctttactt 660
atatctgtga ctgtctgtag tttgctcttg gtccttatca tattattttg ttacttccgg 720
tataaaagac aagaaaccag acctcgatac agcattgggt tagaacagga tgaaacttac 780
attcctcctg gagaatccct gagagactta attgagcagt ctcagagctc aggaagtgga 840
tcaggcctcc ctctgctggt ccaaaggact atagctaagc agattcagat ggtgaaacag 900
attggaaaag gtcgctatgg ggaagtttgg atgggaaagt ggcgtggcga aaaggtagct 960
gtgaaagtgt tcttcaccac agaggaagcc agctggttca gagagacaga aatatatcag 1020
acagtgttga tgaggcatga aaacattttg ggtttcattg ctgcagatat caaagggaca 1080
gggtcctgga cccagttgta cctaatcaca gactatcatg aaaatggttc cctttatgat 1140
tatctgaagt ccaccaccct agacgctaaa tcaatgctga agttagccta ctcttctgtc 1200
agtggcttat gtcatttaca cacagaaatc tttagtactc aaggcaaacc agcaattgcc 1260
catcgagatc tgaaaagtaa aaacattctg gtgaagaaaa atggaacttg ctgtattgct 1320
gacctgggcc tggctgttaa atttattagt gatacaaatg aagttgacat accacctaac 1380
actcgagttg gcaccaaacg ctatatgcct ccagaagtgt tggacgagag cttgaacaga 1440
aatcacttcc agtcttacat catggctgac atgtatagtt ttggcctcat cctttgggag 1500
gttgctagga gatgtgtatc aggaggtata gtggaagaat accagcttcc ttatcatgac 1560
ctagtgccca gtgacccctc ttatgaggac atgagggaga ttgtgtgcat caagaagtta 1620
cgcccctcat tcccaaaccg gtggagcagt gatgagtgtc taaggcagat gggaaaactc 1680
atgacagaat gctgggctca caatcctgca tcaaggctga cagccctgcg ggttaagaaa 1740
acacttgcca aaatgtcaga gtcccaggac attaaactct gataggagag gaaaagtaag 1800
catctctgca gaaagccaac aggtactctt ctgtttgtgg gcagagcaaa agacatcaaa 1860
taagcatcca cagtacaagc cttgaacatc gtcctgcttc ccagtgggtt cagacctcac 1920
ctttcaggga gcgacctggg caaagacaga gaagctccca gaaggagaga ttgatccgtg 1980
tctgtttgta ggcggagaaa ccgttgggta acttgttcaa gatatgatgc at 2032

<210> 40
<211> 502
<212> PRT

<213>  homo sapiens

<400>  

<210>  41
<211>  3611
<212>  DNA
<213>  homo sapiens

<400>  SEQ ID NO:41
cgccccccga ccccggatcg aatccccgcc ctccgcaccc tggatatgtt ttctcccaga      60

cctggatatt tttttgatat cgtgaaacta cgagggaaat aatttggggg atttcttctt 120

ggctccctgc tttccccaca gacatgcctt ccgtttggag ggccgcggca ccccgtccga 180

ggcgaaggaa cccccccagc cgcgagggag agaaatgaag ggaatttctg cagcggcatg 240

aaagctctgc agctaggtcc tctcatcagc catttgtcct ttcaaactgt attgtgatac 300

gggcaggatc agtccacggg agagaagacg agcctcccgg ctgtttctcc gccggtctac 360

ttcccatatt tcttttcttt gccctcctga ttcttggctg gcccagggat gacttcctcg 420

ctgcagcggc cctggcgggt gccctggcta ccatggacca tcctgctggt cagcactgcg 480

gctgcttcgc agaatcaaga acggctatgt gcgtttaaag atccgtatca gcaagacctt 540

gggataggtg agagtagaat ctctcatgaa aatgggacaa tattatgctc gaaaggtagc 600

acctgctatg gcctttggga gaaatcaaaa ggggacataa atcttgtaaa acaaggatgt 660

tggtctcaca ttggagatcc ccaagagtgt cactatgaag aatgtgtagt aactaccact 720

cctccctcaa ttcagaatgg aacataccgt ttctgctgtt gtagcacaga tttatgtaat 780

gtcaacttta ctgagaattt tccacctcct gacacaacac cactcagtcc acctcattca 840

tttaaccgag atgagacaat aatcattgct ttggcatcag tctctgtatt agctgttttg 900

atagttgcct tatgctttgg atacagaatg ttgacaggag accgtaaaca aggtcttcac 960

agtatgaaca tgatggaggc agcagcatcc gaaccctctc ttgatctaga taatctgaaa 1020

ctgttggagc tgattggccg aggtcgatat ggagcagtat ataaaggctc cttggatgag 1080

cgtccagttg ctgtaaaagt gttttccttt gcaaaccgtc agaattttat caacgaaaag 1140

aacatttaca gagtgccttt gatggaacat gacaacattg cccgctttat agttggagat 1200

gagagagtca ctgcagatgg acgcatggaa tatttgcttg tgatggagta ctatcccaat 1260

ggatctttat gcaagtattt aagtctccac acaagtgact gggtaagctc ttgccgtctt 1320

gctcattctg ttactagagg actggcttat cttcacacag aattaccacg aggagatcat 1380

tataaacctg caatttccca tcgagattta aacagcagaa atgtcctagt gaaaaatgat 1440

ggaacctgtg ttattagtga ctttggactg tccatgaggc tgactggaaa tagactggtg 1500

cgcccagggg aggaagataa tgcagccata agcgaggttg gcactatcag atatatggca 1560

ccagaagtgc tagaaggagc tgtgaacttg agggactgtg aatcagcttt gaaacaagta 1620

gacatgtatg ctcttggact aatctattgg gagatattta tgagatgtac agacctcttc 1680

ccaggggaat ccgtaccaga gtaccagatg gcttttcaga cagaggttgg aaaccatccc 1740

acttttgagg atatgcaggt tctcgtgtct agggaaaaac agagacccaa gttcccagaa 1800

gcctggaaag aaaatagcct ggcagtgagg tcactcaagg agacaatcga agactgttgg 1860

gaccaggatg cagaggctcg gcttactgca cagtgtgctg aggaaaggat ggctgaactt 1920

atgatgattt gggaaagaaa caaatctgtg agcccaacag tcaatccaat gtctactgct 1980

atgcagaatg aacgcaacct gtcacataat aggcgtgtgc caaaaattgg tccttatcca 2040

gattattctt cctcctcata cattgaagac tctatccatc atactgacag catcgtgaag 2100

aatatttcct ctgagcattc tatgtccagc acacctttga ctatagggga aaaaaaccga 2160

aattcaatta actatgaacg acagcaagca caagctcgaa tccccagccc tgaaacaagt 2220

gtcaccagcc tctccaccaa cacaacaacc acaaacacca caggactcac gccaagtact 2280

ggcatgacta ctatatctga gatgccatac ccagatgaaa caaatctgca taccacaaat 2340

gttgcacagt caattgggcc aacccctgtc tgcttacagc tgacagaaga agacttggaa 2400

accaacaagc tagacccaaa agaagttgat aagaacctca aggaaagctc tgatgagaat 2460

ctcatggagc actctcttaa acagttcagt ggcccagacc cactgagcag tactagttct 2520

agcttgcttt acccactcat aaaacttgca gtagaagcaa ctggacagca ggacttcaca 2580

cagactgcaa atggccaagc atgtttgatt cctgatgttc tgcctactca gatctatcct 2640

ctccccaagc agcagaacct tcccaagaga cctactagtt tgcctttgaa caccaaaaat 2700

tcaacaaaag agccccggct aaaatttggc agcaagcaca aatcaaactt gaaacaagtc 2760

gaaactggag ttgccaagat gaatacaatc aatgcagcag aacctcatgt ggtgacagtc 2820

accatgaatg gtgtggcagg tagaaaccac agtgttaact cccatgctgc cacaacccaa 2880

tatgccaatg ggacagtact atctggccaa acaaccaaca tagtgacaca tagggcccaa 2940

gaaatgttgc agaatcagtt tattggtgag gacacccggc tgaatattaa ttccagtcct 3000

gatgagcatg agcctttact gagacgagag caacaagctg gccatgatga aggtgttctg 3060

gatcgtcttg tggacaggag ggaacggcca ctagaaggtg gccgaactaa ttccaataac 3120

aacaacagca atccatgttc agaacaagat gttcttgcac agggtgttcc aagcacagca 3180

gcagatcctg ggccatcaaa gcccagaaga gcacagaggc ctaattctct ggatctttca 3240

gccacaaatg tcctggatgg cagcagtata cagataggtg agtcaacaca agatggcaaa 3300

tcaggatcag gtgaaaagat caagaaacgt gtgaaaactc cctattctct taagcggtgg 3360

cgcccctcca cctgggtcat ctccactgaa tcgctggact gtgaagtcaa caataatggc 3420

agtaacaggg cagttcattc caaatccagc actgctgttt accttgcaga aggaggcact 3480

gctacaacca tggtgtctaa agatatagga atgaactgtc tgtgaaatgt tttcaagcct 3540

atggagtgaa attatttttt gcatcattta aacatgcaga agatgtttaa aaataaaaaa 3600

aaaactgctt t 3611

<210> 42
<211> 1038
<212> PRT
<213> homo sapiens

<400> 

<210> 43
<211> 3561
<212> DNA
<213> homo sapiens

<400> SEQ ID NO:43
cccgggtcag cgcccgcccg cccgcgctcc tcccggccgc tcctcccgcc ccgcccggcc 60

cggcgccgac tctgcggccg cccgacgagc ccctcgcggc actgccccgg ccccggcccc 120

ggccccggcc ccctcccgcc gcaccgcccc cggcccggcc ctccgccctc cgcactcccg 180

cctccctccc tccgcccgct cccgcgccct cctccctccc tcctccccag ctgtcccgtt 240

cgcgtcatgc cgagcctccc ggccccgccg gccccgctgc tgctcctcgg gctgctgctg 300

ctcggctccc ggccggcccg cggcgccggc cccgagcccc ccgtgctgcc catccgttct 360

gagaaggagc cgctgcccgt tcggggagcg gcaggctgca ccttcggcgg gaaggtctat 420

gccttggacg agacgtggca cccggaccta ggggagccat tcggggtgat gcgctgcgtg 480

ctgtgcgcct gcgaggcgac agggaccttg aggcccagag agatgaagta gcttgtctag 540

ggtcacgcag cttcctcagt ggggtcgccg taccaggggc cctggcaggg tcagctgcaa 600

gaacatcaaa ccagagtgcc caaccccggc ctgtgggcag ccgcgccagc tgccgggaca 660

ctgctgccag acctgccccc aggagcgcag cagttcggag cggcagccga gcggcctgtc 720

cttcgagtat ccgcgggacc cggagcatcg cagttatagc gaccgcgggg agccaggcgc 780

tgaggagcgg gcccgtggtg acggccacac ggacttcgtg gcgctgctga cagggccgag 840
gtcgcaggcg gtggcacgag cccgagtctc gctgctgcgc tctagcctcc gcttctctat 900
ctcctacagg cggctggacc gccctaccag gatccgcttc tcagactcca atggcagtgt 960
cctgtttgag caccctgcag cccccaccca agatggcctg gtctgtgggg tgtggcgggc 1020
agtgcctcgg ttgtctctgc ggctccttag ggcagaacag ctgcatgtgg cacttgtgac 1080
actcactcac ccttcagggg aggtctgggg gcctctcatc cggcaccggg ccctggctgc 1140
agagaccttc agtgccatcc tgactctaga aggcccccca cagcagggcg tagggggcat 1200
caccctgctc actctcagtg acacagagga ctccttgcat tttttgctgc tcttccgagg 1260
gctgctggaa cccaggagtg gggattctac accaggggca gctactgcga gaacttcagg 1320
ccaatgtctc agcccaggaa ccaggctttg ctgaggtgct gcccaacctg acagtccagg 1380
agatggactg gctggtgctg ggggagctgc agatggccct ggagtgggca ggcaggccag 1440
ggctgcgcat cagtggacac attgctgcca ggaagagctg cgacgtcctg caaagtgtcc 1500
tttgtggggc tgatgccctg atcccagtcc agacgggtgc tgccggctca gccagcctca 1560
cgctgctagg aaatggctcc ctgatctatc aggtgcaagt ggtagggaca agcagtgagg 1620
tggtggccat gacactggag accaagcctc agcggaggga tcagcgcact gtcctgtgcc 1680
acatggctgg actccagcca ggaggacaca cggccgtggg tatctgccct gggctgggtg 1740
cccgaggggc tcatatgctg ctgcagaatg agctcttcct gaacgtgggc accaaggact 1800
tcccagacgg agagcttcgg gggcacgtgg ctgccctgcc ctactgtggg catagctccc 1860
gccatgacac gctgcccgtg cccctagcag gagccctggt gctaccccct gtgaagagcc 1920
aagcagcagg gcacgcctgg ctttccttgg atacccactg tcacctgcac tatgaagtgc 1980
tgctggctgg gcttggtggc tcagaacaag gcactgtcac tgcccacctc cttgggcctc 2040
ctggaacgcc agggcctcgg cggctgctga agggattcta tggctcagag gcccagggtg 2100
tggtgaagga cctggagccg gaactgctgc ggcacctggc aaaaggcatg gcctccctga 2160
tgatcaccac caagggtagc ccagaggggg agctccgagg gcaggtgcac atagccaacc 2220
aatgtgaggt tggcggactg cgcctggagg cggccggggc cgagggggtg cgggcgctgg 2280
gggctccgga tacagcctct gctgcgccgc ctgtggtgcc tggtctcccg gccctagcgc 2340
ccgccaaacc tggtggtcct gggcggcccc gagaccccaa cacatgcttc ttcgaggggc 2400
agcagcgccc ccacggggct cgctgggcgc ccaactacga cccgctctgc tcactctgca 2460
cctgccagag acgaacggtg atctgtgacc cggtggtgtg cccaccgccc agctgcccac 2520
acccggtgca ggctcccgac cagtgctgcc ctgtttgccc tgagaaacaa gatgtcagag 2580
acttgccagg gctgccaagg agccgggacc caggagaggg ctgctatttt gatggtgacc 2640
ggagctggcg ggcagcgggt acgcggtggc accccgttgt gccccccttt ggcttaatta 2700
agtgtgctgt ctgcacctgc aaggggggca ctggagaggt gcactgtgag aaggtgcagt 2760
gtccccggct ggcctgtgcc cagcctgtgc gtgtcaaccc caccgactgc tgcaaacagt 2820
gtccagtggg gtcgggggcc cacccccagc tgggggaccc catgcaggct gatgggcccc 2880

ggggctgccg ttttgctggg cagtggttcc cagagagtca gagctggcac ccctcagtgc 2940

ccccttttgg agagatgagc tgtatcacct gcagatgtgg ggcaggggtg cctcactgtg 3000

agcgggatga ctgttcactg ccactgtcct gtggctcggg gaaggagagt cgatgctgtt 3060

cccgctgcac ggcccaccgg cggccagccc cagagaccag aactgatcca gagctggaga 3120

aagaagccga aggctcttag ggagcagcca gagggccaag tgaccaagag gatggggcct 3180

gagctgggga aggggtggca tcgaggacct tcttgcattc tcctgtggga agcccagtgc 3240

ctttgctccc ctgtcctgcc tctactccca cccccactac ctctgggaac cacagctcca 3300

caagggggag aggcagctgg gccagaccga ggtcacagcc actccaagtc ctgccctgcc 3360

accctcggcc tctgtcctgg aagccccacc cctttcctcc tgtacataat gtcactggct 3420

tgttgggatt tttaatttat cttcactcag caccaagggc ccccgacact ccactcctgc 3480

tgcccctgag ctgagcagag tcattattgg agagttttgt atttattaaa acatttcttt 3540

ttcagtcaaa aaaaaaaaa a 3561

<210> 44
<211> 94
<212> PRT
<213> homo sapiens

<400> SEQ ID NO:44

Met Pro Ser Leu Pro Ala Pro Pro Ala Pro Leu Leu Leu Leu Gly Leu
1               5                   10                  15

Leu Leu Leu Gly Ser Arg Pro Ala Arg Gly Ala Gly Pro Glu Pro Pro
            20                  25                  30

Val Leu Pro Ile Arg Ser Glu Lys Glu Pro Leu Pro Val Arg Gly Ala
        35                  40                  45

Ala Gly Cys Thr Phe Gly Gly Lys Val Tyr Ala Leu Asp Glu Thr Trp
    50                  55                  60

His Pro Asp Leu Gly Glu Pro Phe Gly Val Met Arg Cys Val Leu Cys
65                  70                  75                  80

Ala Cys Glu Ala Thr Gly Thr Leu Arg Pro Arg Glu Met Lys
                85                  90

<210> 45
<211> 4049
<212> DNA
<213> homo sapiens

<400> SEQ ID NO:45
gcggccgcac tcagcgccac gcgtcgaaag cgcaggcccc gaggacccgc cgcactgaca 60

gtatgagccg cacagcctac acggtgggag ccctgcttct cctcttgggg accctgctgc 120

cggctgctga agggaaaaag aaagggtccc aaggtgccat ccccccgcca gacaaggccc 180

agcacaatga ctcagagcag actcagtcgc cccagcagcc tggctccagg aaccgggggc 240

ggggccaagg gcggggcact gccatgcccg gggaggaggt gctggagtcc agccaagagg 300
ccctgcatgt gacggagcgc aaatacctga agcgagactg gtgcaaaacc cagccgctta 360
agcagaccat ccacgaggaa ggctgcaaca gtcgcaccat catcaaccgc ttctgttacg 420
gccagtgcaa ctctttctac atccccaggc acatccggaa ggaggaaggt tcctttcagt 480
cctgctcctt ctgcaagccc aagaaattca ctaccatgat ggtcacactc aactgccctg 540
aactacagcc acctaccaag aagaagagag tcacacgtgt gaagcagtgt cgttgcatat 600
ccatcgattt ggattaagcc aaatccaggt gcacccagca tgtcctagga atgcagcccc 660
aggaagtccc agacctaaaa caaccagatt cttacttggc ttaaacctag aggccagaag 720
aacccccagc tgcctcctgg caggagcctg cttgtgcgta gttcgtgtgc atgagtgtgg 780
atgggtgcct gtgggtgttt ttagacacca gagaaaacac agtctctgct agagagcact 840
ccctattttg taaacatatc tgctttaatg gggatgtacc agaaacccac ctcaccccgg 900
ctcacatcta aggggcgggg ccgtggtctg gttctgactt tgtgtttttg tgccctcctg 960
gggaccagaa tctcctttcg gaatgaatgt tcatggaaga ggctcctctg agggcaagag 1020
acctgtttta gtgctgcatt cgacatggaa aagtcctttt aacctgtgct tgcatcctcc 1080
tttcctcctc ctcctcacaa tccatctctt cttaagttga tagtgactat gtcagtctaa 1140
tctcttgttt gccaaggttc ctaaattaat tcacttaacc atgatgcaaa tgtttttcat 1200
tttgtgaaga ccctccagac tctgggagag gctggtgtgg gcaaggacaa gcaggatagt 1260
ggagtgagaa agggagggtg gagggtgagg ccaaatcagg tccagcaaaa gtcagtaggg 1320
acattgcaga agcttgaaag gccaataccc agaacacagg ctgatgcttc tgagaaagtc 1380
ttttcctagt atttaacaga acccaagtga acagaggaga aatgagattg ccagaaagtg 1440
attaactttg gccgttgcaa tctgctcaaa cctaacacca aactgaaaac ataaatactg 1500
accactccta tgttcggacc caagcaagtt agctaaacca aaccaactcc tctgctttgt 1560
ccctcaggtg gaaaagagag gtagtttaga actctctgca taggggtggg aattaatcaa 1620
aaacckcaga ggctgaaatt cctaatacct ttcctttatc gtggttatag tcagctcatt 1680
tccattccac tatttcccat aatgcttctg agagccacta acttgattga taaagatcct 1740
gcctctgctg agtgtacctg acagtaagtc taaagatgar agagtttagg gactactctg 1800
ttttagcaag aratattktg gggtcttttt tgttttaact attgtcagga gattgggcta 1860
ragagaagac gacgagagta aggaaataaa gggrattgcc tctggctaga gagtaagtta 1920
ggtgttaata cctggtagaa atgtaaggga tatgacctcc ctttctttat gtgctcactg 1980
aggatctgag gggaccctgt taggagagca tagcatcatg atgtattagc tgttcatctg 2040
ctactggttg gatggacata actattgtaa ctattcagta tttactggta ggcactgtcc 2100
tctgattaaa cttggcctac tggcaatggc tacttaggat tgatctaagg gccaaagtgc 2160
agggtgggtg aactttattg tactttggat ttggttaacc tgttttcttc aagcctgagg 2220
ttttatatac aaactccctg aatactcttt ttgccttgta tcttctcagc ctcctagcca 2280
agtcctatgt aatatggaaa acaaacactg cagacttgag attcagttgc cgatcaaggc 2340

tctggcattc agagaaccct tgcaactcga gaagctgttt ttatttcgtt tttgttttga 2400

tccagtgctc tcccatctaa caactaaaca ggagccattt caaggcggga gatattttaa 2460

acacccaaaa tgttgggtct gattttcaaa cttttaaact cactactgat gattctcacg 2520

ctaggcgaat ttgtccaaac acatagtgtg tgtgttttgt atacactgta tgaccccacc 2580

ccaaatcttt gtattgtcca cattctccaa caataaagca cagagtggat ttaattaagc 2640

acacaaatgc taaggcagaa ttttgagggt gggagagaag aaaagggaaa gaagctgaaa 2700

atgtaaaacc acaccaggga ggaaaaatga cattcagaac cagcaaacac tgaatttctc 2760

ttgttgtttt aactctgcca caagaatgca atttcgttaa tggagatgac ttaagttggc 2820

agcagtaatc ttcttttagg agcttgtacc acagtcttgc acataagtgc agatttggct 2880

caagtaaaga gaatttcctc aacactaact tcactgggat aatcagcagc gtaactaccc 2940

taaaagcata tcactagcca aagagggaaa tatctgttct tcttactgtg cctatattaa 3000

gactagtaca aatgtggtgt gtcttccaac tttcattgaa aatgccatat ctataccata 3060

ttttattcga gtcactgatg atgtaatgat atattttttc attattatag tagaatattt 3120

ttatggcaag atatttgtgg tcttgatcat acctattaaa ataatgccaa acaccaaata 3180

tgaattttat gatgtacact ttgtgcttgg cattaaaaga aaaaaacaca catcctggaa 3240

gtctgtaagt tgttttttgt tactgtaggt cttcaaagtt aagagtgtaa gtgaaaaatc 3300

tggaggagag gataatttcc actgtgtgga atgtgaatag ttaaatgaaa agttatggtt 3360

atttaatgta attattactt caaatccttt ggtcactgtg atttcaagca tgttttcttt 3420

ttctccttta tatgactttc tctgagttgg gcaaagaaga agctgacaca ccgtatgttg 3480

ttagagtctt ttatctggtc aggggaaaca aaatcttgac ccagctgaac atgtcttcct 3540

gagtcagtgc ctgaatcttt attttttaaa ttgaatgttc cttaaaggtt aacatttcta 3600

aagcaatatt aagaaagact ttaaatgtta ttttggaaga cttacgatgc atgtatacaa 3660

acgaatagca gataatgatg actagttcac acataaagtc cttttaagga gaaaatctaa 3720

aatgaaaagt ggataaacag aacatttata agtgatcagt taatgcctaa gagtgaaagt 3780

agttctattg acattcctca agatatttaa tatcaactgc attatgtatt atgtctgctt 3840

aaatcattta aaaacggcaa agaattatat agactatgag gtaccttgct gtgtaggagg 3900

atgaaagggg agttgatagt ctcataaaac taatttggct tcaagtttca tgaatctgta 3960

actagaattt aattttcacc ccaataatgt tctatatagc ctttgctaaa gagcaactaa 4020

taaattaaac ctattctttc aaaaaaaaa 4049

<210> 46
<211> 184
<212> PRT
<213> homo sapiens

<400> SEQ ID NO:46

Met Ser Arg Thr Ala Tyr Thr Val Gly Ala Leu Leu Leu Leu Leu Gly
1               5                   10                  15

Thr Leu Leu Pro Ala Ala Glu Gly Lys Lys Lys Gly Ser Gln Gly Ala
            20                  25                  30

Ile Pro Pro Pro Asp Lys Ala Gln His Asn Asp Ser Glu Gln Thr Gln
        35                  40                  45

Ser Pro Gln Gln Pro Gly Ser Arg Asn Arg Gly Arg Gly Gln Gly Arg
    50                  55                  60

Gly Thr Ala Met Pro Gly Glu Glu Val Leu Glu Ser Ser Gln Glu Ala
65                  70                  75                  80

Leu His Val Thr Glu Arg Lys Tyr Leu Lys Arg Asp Trp Cys Lys Thr
                85                  90                  95

Gln Pro Leu Lys Gln Thr Ile His Glu Glu Gly Cys Asn Ser Arg Thr
            100                 105                 110

Ile Ile Asn Arg Phe Cys Tyr Gly Gln Cys Asn Ser Phe Tyr Ile Pro
        115                 120                 125

Arg His Ile Arg Lys Glu Glu Gly Ser Phe Gln Ser Cys Ser Phe Cys
    130                 135                 140

Lys Pro Lys Lys Phe Thr Thr Met Met Val Thr Leu Asn Cys Pro Glu
145                 150                 155                 160

Leu Gln Pro Pro Thr Lys Lys Lys Arg Val Thr Arg Val Lys Gln Cys
                165                 170                 175

Arg Cys Ile Ser Ile Asp Leu Asp
            180

<210>  47
<211>  1386
<212>  DNA
<213>  homo sapiens

<400>  SEQ ID NO:47
gctcctcgcc ccgcgcctgc ccccaggatg gtccgcgcga ggcaccagcc gggtgggctt      60

tgcctcctgc tgctgctgct ctgccagttc atggaggacc gcagtgccca ggctgggaac     120

tgctggctcc gtcaagcgaa gaacggccgc tgccaggtcc tgtacaagac cgaactgagc     180

aaggaggagt gctgcagcac cggccggctg agcacctcgt ggaccgagga ggacgtgaat     240

gacaacacac tcttcaagtg gatgattttc aacgggggcg cccccaactg catcccctgt     300

aaagaaacgt gtgagaacgt ggactgtgga cctgggaaaa aatgccgaat gaacaagaag     360

aacaaacccc gctgcgtctg cgccccggat tgttccaaca tcacctggaa gggtccagtc     420

tgcgggctgg atgggaaaac ctaccgcaat gaatgtgcac tcctaaaggc aagatgtaaa     480

gagcagccag aactggaagt ccagtaccaa ggcagatgta aaaagacttg tcgggatgtt     540

ttctgtccag gcagctccac atgtgtggtg gaccagacca ataatgccta ctgtgtgacc     600

tgtaatcgga tttgcccaga gcctgcttcc tctgagcaat atctctgtgg gaatgatgga 660
gtcacctact ccagtgcctg ccacctgaga aaggctacct gcctgctggg cagatctatt 720
ggattagcct atgagggaaa gtgtatcaaa gcaaagtcct gtgaagatat ccagtgcact 780
ggtgggaaaa aatgtttatg ggatttcaag gttgggagag gccggtgttc cctctgtgat 840
gagctgtgcc ctgacagtaa gtcggatgag cctgtctgtg ccagtgacaa tgccacttat 900
gccagcgagt gtgccatgaa ggaagctgcc tgctcctcag gtgtgctact ggaagtaaag 960
cactccggat cttgcaactg aatctgcccg taaaacctga gccattgatt cttcagaact 1020
ttctgcagtt tttgacttca tagattatgc tttaaaaaat tttttttaac ttattgcata 1080
acagcagatg ccaaaaacaa aaaaagcatc tcactgcaag tcacataaaa atgcaacgct 1140
gtaatatggc tgtatcagag ggctttgaaa acatacactg agctgcttct gcgctgttgt 1200
tgtccgtatt taaacaacag ctcccctgta ttcccccatc tagccatttc ggaagacacc 1260
gaggaagagg aggaagatga agaccaggac tacagctttc ctatatcttc tattctagag 1320
tggtaaactc tctataagtg ttcagtgttc acatagcctt tgtgcaaaaa aaaaaaaaaa 1380
aaaaaa 1386

<210> 48
<211> 317
<212> PRT
<213> homo sapiens

<400> SEQ ID NO:48

Met Val Arg Ala Arg His Gln Pro Gly Gly Leu Cys Leu Leu Leu Leu
1               5                   10                  15

Leu Leu Cys Gln Phe Met Glu Asp Arg Ser Ala Gln Ala Gly Asn Cys
            20                  25                  30

Trp Leu Arg Gln Ala Lys Asn Gly Arg Cys Gln Val Leu Tyr Lys Thr
        35                  40                  45

Glu Leu Ser Lys Glu Glu Cys Cys Ser Thr Gly Arg Leu Ser Thr Ser
    50                  55                  60

Trp Thr Glu Glu Asp Val Asn Asp Asn Thr Leu Phe Lys Trp Met Ile
65                  70                  75                  80

Phe Asn Gly Gly Ala Pro Asn Cys Ile Pro Cys Lys Glu Thr Cys Glu
                85                  90                  95

Asn Val Asp Cys Gly Pro Gly Lys Lys Cys Arg Met Asn Lys Lys Asn
            100                 105                 110

Lys Pro Arg Cys Val Cys Ala Pro Asp Cys Ser Asn Ile Thr Trp Lys
        115                 120                 125

Gly Pro Val Cys Gly Leu Asp Gly Lys Thr Tyr Arg Asn Glu Cys Ala
    130                 135                 140

Leu Leu Lys Ala Arg Cys Lys Glu Gln Pro Glu Leu Glu Val Gln Tyr
145                 150                 155                 160

Gln Gly Arg Cys Lys Lys Thr Cys Arg Asp Val Phe Cys Pro Gly Ser
                165                 170                 175

Ser Thr Cys Val Val Asp Gln Thr Asn Asn Ala Tyr Cys Val Thr Cys
            180                 185                 190

Asn Arg Ile Cys Pro Glu Pro Ala Ser Ser Glu Gln Tyr Leu Cys Gly
        195                 200                 205

Asn Asp Gly Val Thr Tyr Ser Ser Ala Cys His Leu Arg Lys Ala Thr
    210                 215                 220

Cys Leu Leu Gly Arg Ser Ile Gly Leu Ala Tyr Glu Gly Lys Cys Ile
225                 230                 235                 240

Lys Ala Lys Ser Cys Glu Asp Ile Gln Cys Thr Gly Gly Lys Lys Cys
                245                 250                 255

Leu Trp Asp Phe Lys Val Gly Arg Gly Arg Cys Ser Leu Cys Asp Glu
            260                 265                 270

Leu Cys Pro Asp Ser Lys Ser Asp Glu Pro Val Cys Ala Ser Asp Asn
        275                 280                 285

Ala Thr Tyr Ala Ser Glu Cys Ala Met Lys Glu Ala Ala Cys Ser Ser
    290                 295                 300

Gly Val Leu Leu Glu Val Lys His Ser Gly Ser Cys Asn
305                 310                 315

<210>  49
<211>  699
<212>  DNA
<213>  homo sapiens

<400>  SEQ ID NO:49
atggagcgct gccccagcct aggggtcacc ctctacgccc tggtggtggt cctggggctg      60

cgggcgacac cggccggcgg ccagcactat ctccacatcc gcccggcacc cagcgacaac     120

ctgcccctgg tggacctcat cgaacaccca gaccctatct ttgaccccaa ggaaaaggat     180

ctgaacgaga cgctgctgcg ctcgctgctc gggggccact acgacccagg cttcatggcc     240

acctcgcccc ccgaggaccg gcccggcggg ggcgggggtg cagctggggg cgcggaggac     300

ctggcggagc tggaccagct gctgcggcag cggccgtcgg gggccatgcc gagcgagatc     360

aaagggctag agttctccga gggcttggcc cagggcaaga agcagcgcct aagcaagaag     420

ctgcggagga agttacagat gtggctgtgg tcgcagacat tctgccccgt gctgtacgcg     480

tggaacgacc tgggcagccg cttttggccg cgctacgtga aggtgggcag ctgcttcagt     540

aagcgctcgt gctccgtgcc cgagggcatg gtgtgcaagc cgtccaagtc cgtgcacctc 600

acggtgctgc ggtggcgctg tcagcggcgc gggggccagc gctgcggctg gattcccatc 660

cagtacccca tcatttccga gtgcaagtgc tcgtgctag 699

<210> 50
<211> 232
<212> PRT
<213> homo sapiens

<400> 

<210>  51
<211>  804
<212>  DNA
<213>  homo sapiens

<400>  SEQ ID NO:51
atgcatctcc tcttatttca gctgctggta ctcctgcctc taggaaagac cacacggcac      60

caggatggcc gccagaatca gagttctctt tcccccgtac tcctgccaag gaatcaaaga     120

gagcttccca caggcaacca tgaggaagct gaggagaagc cagatctgtt tgtcgcagtg     180

ccacaccttg tagccaccag ccctgcaggg gaaggccaga ggcagagaga gaagatgctg     240

tccagatttg gcaggttctg gaagaagcct gagagagaaa tgcatccatc cagggactca     300

gatagtgagc ccttcccacc tgggacccag tccctcatcc agccgataga tggaatgaaa     360

atggagaaat ctcctcttcg ggaagaagcc aagaaattct ggcaccactt catgttcaga     420

aaaactccgg cttctcaggg ggtcatcttg cccatcaaaa gccatgaagt acattgggag     480

acctgcagga cagtgccctt cagccagact ataacccacg aaggctgtga aaaagtagtt     540

gttcagaaca acctttgctt tgggaaatgc gggtctgttc attttcctgg agccgcgcag     600

cactcccata cctcctgctc tcactgtttg cctgccaagt tcaccacgat gcacttgcca     660

ctgaactgca ctgaactttc ctccgtgatc aaggtggtga tgctggtgga ggagtgccag     720

tgcaaggtga agacggagca tgaagatgga cacatcctac atgctggctc ccaggattcc     780

tttatcccag gagtttcagc ttga                                            804

<210>  52
<211>  267
<212>  PRT
<213>  homo sapiens

<400>  SEQ ID NO:52

Met His Leu Leu Leu Phe Gln Leu Leu Val Leu Leu Pro Leu Gly Lys
1               5                   10                  15

Thr Thr Arg His Gln Asp Gly Arg Gln Asn Gln Ser Ser Leu Ser Pro
            20                  25                  30

Val Leu Leu Pro Arg Asn Gln Arg Glu Leu Pro Thr Gly Asn His Glu
        35                  40                  45

Glu Ala Glu Glu Lys Pro Asp Leu Phe Val Ala Val Pro His Leu Val
    50                  55                  60

Ala Thr Ser Pro Ala Gly Glu Gly Gln Arg Gln Arg Glu Lys Met Leu
65                  70                  75                  80

Ser Arg Phe Gly Arg Phe Trp Lys Lys Pro Glu Arg Glu Met His Pro
                85                  90                  95

Ser Arg Asp Ser Asp Ser Glu Pro Phe Pro Pro Gly Thr Gln Ser Leu
            100                 105                 110

Ile Gln Pro Ile Asp Gly Met Lys Met Glu Lys Ser Pro Leu Arg Glu
        115                 120                 125

Glu Ala Lys Lys Phe Trp His His Phe Met Phe Arg Lys Thr Pro Ala
    130                 135                 140

Ser Gln Gly Val Ile Leu Pro Ile Lys Ser His Glu Val His Trp Glu
145                 150                 155                 160

Thr Cys Arg Thr Val Pro Phe Ser Gln Thr Ile Thr His Glu Gly Cys
                165                 170                 175

Glu Lys Val Val Val Gln Asn Asn Leu Cys Phe Gly Lys Cys Gly Ser
            180                 185                 190

Val His Phe Pro Gly Ala Ala Gln His Ser His Thr Ser Cys Ser His
        195                 200                 205

Cys Leu Pro Ala Lys Phe Thr Thr Met His Leu Pro Leu Asn Cys Thr
    210                 215                 220

Glu Leu Ser Ser Val Ile Lys Val Val Met Leu Val Glu Glu Cys Gln
225                 230                 235                 240

Cys Lys Val Lys Thr Glu His Glu Asp Gly His Ile Leu His Ala Gly
                245                 250                 255

Ser Gln Asp Ser Phe Ile Pro Gly Val Ser Ala
            260                 265

<210>  53
<211>  1523
<212>  DNA
<213>  homo sapiens

<400>  SEQ ID NO:53
ctggcgcggg cgggagctgc ggcggatacc cttgcgtgct gtggagaccc tactctcttc      60

gctgagaacg gccgctagcg gggactgaag gccgggagcc cactcccgac ccggggctag     120

cgtgcgtccc tagagtcgag cggggcaagg gagccagtgg ccgccgacgg gggaccggga     180

aacttttctg ggctcctggg cgcgccctgt agccgcgctc catgctccgg cagcggcccg     240

aaacccagcc ccgccgctga cggcgcccgc cgctccgggc agggcccatg ccctgcgcgc     300

tccgggggtc gtaggctgcc gccgagccgg ggctccggaa gccggcgggg gcgccgcggc     360

cgtgcggggc gtcaatggat cgccactcca gctacatctt catctggctg cagctggagc     420

tctgcgccat ggccgtgctg ctcaccaaag gtgaaattcg atgctactgt gatgctgccc     480

actgtgtagc cactggttat atgtgtaaat ctgagctcag cgcctgcttc tctagacttc     540

ttgatcctca gaactcaaat tccccactca cccatggctg cctggactct cttgcaagca     600

cgacagacat ctgccaagcc aaacaggccc gaaaccactc tggcaccacc atacccacat 660

tggaatgctg tcatgaagac atgtgcaatt acagagggct gcacgatgtt ctctctcctc 720

ccaggggtga ggcctcagga caaggaaaca ggtatcagca tgatggtagc agaaacctta 780

tcaccaaggt gcaggagctg acttcttcca aagagttgtg gttccgggca gcggtcattg 840

ccgtgcccat tgctggaggg ctgattttag tgttgcttat tatgttggcc ctgaggatgc 900

ttcgaagtga aaataagagg ctgcaggatc agcggcaaca gatgctctcc cgtttgcact 960

acagctttca cggacaccat tccaaaaagg ggcaggttgc aaagttagac ttggaatgca 1020

tggtgccggt cagtgggcac gagaactgct gtctgacctg tgataaaatg agacaagcag 1080

acctcagcaa cgataagatc ctctcgcttg ttcactgggg catgtacagt gggcacggga 1140

agctggaatt cgtatgacgg agtcttatct gaactacact tactgaacag cttgaaggcc 1200

ttttgagttc tgctggacag gagcacttta tctgaagaca aactcattta atcatctttg 1260

agagacaaaa tgacctctgc aaacagaatc ttggatattt cttctgaagg attatttgca 1320

cagacttaaa tacagttaaa tgtgttattt gcttttaaaa ttataaaaag caaagagaag 1380

actttgtaca cactgtcacc agggttattt gcatccaagg gagctggaat tgagtaccta 1440

aataaacaaa aatgtgccct atgtaagctt ctacatcttg atttattgta aagatttaaa 1500

agaaatatat atattttgtc tga 1523

<210> 54
<211> 260
<212> PRT
<213> homo sapiens

<400> SEQ ID NO:54

Met Asp Arg His Ser Ser Tyr Ile Phe Ile Trp Leu Gln Leu Glu Leu
1               5                   10                  15

Cys Ala Met Ala Val Leu Leu Thr Lys Gly Glu Ile Arg Cys Tyr Cys
            20                  25                  30

Asp Ala Ala His Cys Val Ala Thr Gly Tyr Met Cys Lys Ser Glu Leu
        35                  40                  45

Ser Ala Cys Phe Ser Arg Leu Leu Asp Pro Gln Asn Ser Asn Ser Pro
    50                  55                  60

Leu Thr His Gly Cys Leu Asp Ser Leu Ala Ser Thr Thr Asp Ile Cys
65                  70                  75                  80

Gln Ala Lys Gln Ala Arg Asn His Ser Gly Thr Thr Ile Pro Thr Leu
                85                  90                  95

Glu Cys Cys His Glu Asp Met Cys Asn Tyr Arg Gly Leu His Asp Val
            100                 105                 110

Leu Ser Pro Pro Arg Gly Glu Ala Ser Gly Gln Gly Asn Arg Tyr Gln
        115                 120                 125

His Asp Gly Ser Arg Asn Leu Ile Thr Lys Val Gln Glu Leu Thr Ser
    130                 135                 140

Ser Lys Glu Leu Trp Phe Arg Ala Ala Val Ile Ala Val Pro Ile Ala
145                 150                 155                 160

Gly Gly Leu Ile Leu Val Leu Leu Ile Met Leu Ala Leu Arg Met Leu
                165                 170                 175

Arg Ser Glu Asn Lys Arg Leu Gln Asp Gln Arg Gln Gln Met Leu Ser
            180                 185                 190

Arg Leu His Tyr Ser Phe His Gly His His Ser Lys Lys Gly Gln Val
        195                 200                 205

Ala Lys Leu Asp Leu Glu Cys Met Val Pro Val Ser Gly His Glu Asn
    210                 215                 220

Cys Cys Leu Thr Cys Asp Lys Met Arg Gln Ala Asp Leu Ser Asn Asp
225                 230                 235                 240

Lys Ile Leu Ser Leu Val His Trp Gly Met Tyr Ser Gly His Gly Lys
                245                 250                 255

Leu Glu Phe Val
            260

## Claims

1. A method for identifying an agent useful for the treatment of glaucoma, said method comprising:
a) obtaining a first composition comprising a population of cells expressing BMP-2A, BMP4, BMP-5, or BMP7;
b) obtaining a candidate substance;
c) incubating said composition and said candidate substance;
d) testing said composition for its ability to turn on BMP-induced Smad signaling pathways or BMP-regulated gene expression; and
e) identifying a candidate substance that inhibits, or stimulates, BMP-induced Smad signaling pathways or BMP-regulated gene expression.

2. A method as claimed in Claim 1 in which the cells are human Optic Nerve Head (ONH) cells.

3. A method as claimed in Claim 1 in which the cells are human Trabecular Meshwork (TM) cells.

4. A method as claimed in Claim 1 in which the cells are human Lamina Cribrosa (LC) cells.

5. A method as claimed in any of Claims 1 to 4 in which the cells are recombinant cells.

6. A method as claimed in any preceding Claim in which the testing of step d) employs a method selected from the group comprising: screening for BMP-induced genes; and quantitating levels of mRNA using RT-PCR, DNA microarrays, or reporter gene constructs.
